# EUROPEAN PATENT APPLICATION

(11) **EP 4 548 758 A1**
(43) Date of publication of application: **07.05.2025**
(21) Application number: 24828714.6
(22) Date of filing: 01.04.2024
(51) Int. Cl.: A01K 67/027, C12N 15/85, C12N 5/10, C07K 16/18, C07K 16/46, C12N 5/20

(54) **GENETICALLY MODIFIED MOUSE FOR ANTIBODY PREPARATION AND PREPARATION METHOD THEREFOR**

(30) Priority: 15.09.2023 WO PCT/CN2023/119040
(71) Applicant: Cyagen Biosciences (Suzhou) Inc., Suzhou, Jiangsu 215000 (CN)
(72) Inventor: HAN, Lanqing, Suzhou, Jiangsu 215000 (CN); SU, Cui, Suzhou, Jiangsu 215000 (CN); MOU, Xing, Suzhou, Jiangsu 215000 (CN); WANG, Kang, Suzhou, Jiangsu 215000 (CN); LI, Xiaoliang, Suzhou, Jiangsu 215000 (CN); ZHAI, Xiaochen, Suzhou, Jiangsu 215000 (CN); ZHANG, Liang, Suzhou, Jiangsu 215000 (CN); CHEN, Xiangyun, Suzhou, Jiangsu 215000 (CN); ZHOU, Shun, Suzhou, Jiangsu 215000 (CN)
(74) Representative: Sun, Yiming
(86) International application number: PCT/CN2024/085213
(87) International publication number: WO 2025/055311

(57) **Abstract**

Disclosed is a genetically modified mouse having an immunoglobulin heavy chain locus engineered to insert a gene segment of a human immunoglobulin heavy chain variable region. The mouse is able to reproduce normally and produce a human-mouse chimeric antibody comprising a human heavy chain variable region and a mouse constant region. Also provided is a method for preparing the genetically modified mouse and use of the mouse.

## Description

### TECHNICAL FIELD

The present invention relates to a genetically modified mouse, a cell, an embryo, and a tissue, in particular to a mouse with a humanized immunoglobulin heavy chain variable region genome and a preparation method therefor. The present invention also relates to a genome of the modified mouse, a cell and tissue comprising the genome, and a method for preparing a monoclonal antibody by using the mouse and use thereof. The present invention also relates to a mouse having the modified genome.

### BACKGROUND

Humanized mice exhibit a fully functional humoral immune system that is essentially indistinguishable from that of wild-type mice, show normal cell populations at all stages of B cell development, and exhibit normal lymphoid organ morphology. Antibody sequences of the mice exhibit normal V(D)J rearrangement and normal somatic hypermutation frequency. Antibody populations in these mice reflect isotype distributions that result from normal isotype switching (e.g., normal isotype cis-switching). Immunization of mice results in potent humoral immune responses that generate a large diversity of antibodies having human immunoglobulin variable regions suitable as therapeutic candidates.

The precise replacement of mouse immunoglobulin variable sequences with human immunoglobulin variable sequences allows the formation of genetically modified or transgenic mice. However, due to the divergent evolution of immunoglobulin loci between mice and humans, even a precise replacement of endogenous mouse immunoglobulin sequences at heavy and light chain loci with corresponding human immunoglobulin sequences, by sequential recombineering of very large segments of human immunoglobulin sequences, may also present certain problems. For example, intergenic sequences interspersed within the immunoglobulin loci are inconsistent between mice and humans and may not be functionally equivalent in some cases. Differences in the immunoglobulin loci between mice and humans may still lead to abnormalities in humanized mice, particularly when certain portions of endogenous mouse immunoglobulin heavy chain loci are humanized or otherwise manipulated. Some modifications at the mouse immunoglobulin heavy chain loci are detrimental, such as loss of the ability of the modified mouse to mate and produce offspring. It has been found that the decline or disappearance of fertility in male mice is associated with an impairment of an Adam6 gene.

The ADAM6 protein is a member of the ADAM family of proteins, where ADAM is an acronym for A disintegrin and metalloprotease. The large and diverse ADAM family has a variety of functions including cell adhesion. Some members of the ADAM family are involved in spermatogenesis and fertilization. For example, ADAM2 encodes a subunit of the protein fertilizin, which is involved in sperm-egg interactions. ADAM3 or cyritestin appears to be essential for the binding of sperms to zona pellucida. The absence of ADAM2 or ADAM3 both results in infertility. It has been hypothesized that ADAM2, ADAM3, and ADAM6 form a complex on the surface of mouse sperm cells. The human ADAM6 gene is located between the human VH genes VH1-2 and VH6-1. In mice, there are two kinds of ADAM6 genes, ADAM6a and ADAM6b, present in an intergenic region between mouse VH and DH gene segments, and in mice, the transcriptional orientation of the ADAM6a and ADAM6b genes is opposite to that of the surrounding immunoglobulin gene segments.

CN105861548B discloses an ADAM6 mouse, wherein one or more human immunoglobulin gene sequences are inserted into an immunoglobulin heavy chain locus in the germline of the mouse, and the insertion disrupts the function of endogenous ADAM6 genes; a nucleic acid sequence encoding a mouse ADAM6a protein and a nucleic acid sequence encoding a mouse ADAM6b protein are then inserted into the germline of the mouse, the mouse ADAM6a protein and the mouse ADAM6b protein are expressed from the nucleic acid sequences, and the mouse ADAM6a protein and the mouse ADAM6b protein are expressed from the nucleic acid sequences and improve or restore fertility when expressed in male mice. The disruption of the endogenous ADAM6 genes is undesirable, which may affect the long-term reproductive capacity of mice.

In view of the above, the present invention provides a mouse genome with a modified heavy chain locus, which retains the endogenous ADAM6 genes and their functions, and a human-mouse chimeric antibody can be produced while the fertility is maintained.

### SUMMARY

One aspect of the present invention provides a method for preparing a genetically modified mouse, the method comprising:
(a) inserting a first partial segment of a human immunoglobulin heavy chain variable region locus between an mIgHJ region and an mIgHC region of an immunoglobulin heavy chain locus of a first mouse, wherein the first partial segment comprises a first partial hIgHV contiguous segment, an entire hIgHD segment, and an entire hIgHJ segment, the first partial segment does not comprise a segment between downstream of hIgHV1-2 gene and upstream of higHV6-1 gene, and a first recombination site exists between upstream of the first partial segment and downstream of the mIgHJ region;
(b) inserting a second partial segment of the human immunoglobulin heavy chain variable region locus between an mIgHJ region and an mIgHC region of an immunoglobulin heavy chain locus of a second mouse, wherein the second partial segment is located upstream of the first partial segment, the second partial segment comprises a second partial hIgHV contiguous segment, and a second recombination site exists between downstream of the second partial hIgHV contiguous segment and upstream of the mIgHC region;
(c) hybridizing the first mouse with the second mouse, and screening to obtain a third mouse, wherein the second partial segment and the first partial segment located downstream of the second partial segment of the human immunoglobulin heavy chain variable region locus are inserted between an mIgHJ region and an mIgHC region of an immunoglobulin heavy chain locus of the third mouse, and a third recombination site exists between the second partial segment and the first partial segment; and
(d) knocking out mIgHV5-1 gene of the immunoglobulin heavy chain locus of the third mouse and an entire mIgHV segment upstream thereof, a contiguous segment between mIgHD1-1 gene and mIgHJ4 gene, and mIgHD3-1, migHD5-1, and mIgHD1-3 genes to obtain the genetically modified mouse.

In the present invention, the first partial hIgHV contiguous segment of the first partial segment may start from any gene (including a functional gene, pseudogene, or ORF) in the hIgHV segment upstream of the higHV1-2 gene and terminate at the higHV1-2 gene.

In one embodiment, any gene in the hIgHV segment upstream of the hIgHV1-2 gene may be any functional gene, pseudogene, or ORF between hIgHV(III)-82 and hIgHV1-2 genes. In one embodiment, any functional gene in the hIgHV segment upstream of the higHV1-2 gene is, for example, hIgHV3-74, higHV3-73, higHV3-72, higHV2-70, higHV1-69D, higHV1-69-2, higHV2-70D, higHV1-69, higHV3-66, higHV3-64, higHV3-62, higHV4-61, higHV4-59, higHV1-58, higHV3-53, higHV5-51, higHV3-49, higHV3-48, higHV1-46, higHV1-45, higHV3-43, higHV4-39, higHV3-43D, higHV4-38-2, higHV3-35, higHV4-34, higHV3-33, higHV4-31, higHV3-30-5, higHV4-30-4, hIgHV3-30-3, higHV4-30-2, higHV4-30-1, higHV3-30, higHV4-28, higHV2-26, higHV1-24, higHV3-23D, higHV3-23, higHV3-21, higHV3-20, higHV1-18, higHV3-15, higHV3-13, higHV3-11, hIgHV5-10-1, higHV3-9, higHV3-64D, higHV3-8, higHV3-7, higHV2-5, hIgHV7-4-1, higHV4-4, or hIgHV1-3.

In one embodiment, the first partial hIgHV contiguous segment of the first partial segment starts from one of higHV3-74, higHV3-73, higHV3-72, higHV2-70, higHV1-69D, higHV1-69-2, higHV2-70D, higHV1-69, higHV3-66, higHV3-64, hIgHV3-62, higHV4-61, higHV4-59, higHV1-58, higHV3-53, higHV5-51, higHV3-49, higHV3-48, higHV1-46, higHV1-45, higHV3-43, higHV4-39, higHV3-43D, higHV4-38-2, higHV3-35, higHV4-34, higHV3-33, higHV4-31, higHV3-30-5, higHV4-30-4, higHV3-30-3, higHV4-30-2, higHV4-30-1, higHV3-30, higHV4-28, higHV2-26, higHV1-24, higHV3-23D, higHV3-23, higHV3-21, higHV3-20, higHV1-18, higHV3-15, higHV3-13, higHV3-11, hIgHV5-10-1, higHV3-9, higHV3-64D, higHV3-8, higHV3-7, higHV2-5, hIgHV7-4-1, higHV4-4, or higHV1-3, and terminates at the higHV1-2 gene, and the described genes are both contained as ends. In one embodiment, the first partial hIgHV contiguous segment of the first partial segment is a contiguous segment between the hIgHV4-28 and hIgHV1-2 genes.

In the present invention, the second partial segment is located upstream of the first partial segment, and the second partial hIgHV contiguous segment may start from any gene (including a functional gene, pseudogene, or ORF) in the hIgHV segment and terminate at any gene (including a functional gene, pseudogene, or ORF) in the hIgHV segment upstream of the start gene of the first partial hIgHV contiguous segment. In some embodiments, the second partial segment, together with the first partial segment, constitutes all functional genes upstream of hIgHV1-2 and contiguous segments therebetween, i.e., contiguous segments between the higHV3-74 and higHV1-2 genes. For example, in some embodiments, the second partial hIgHV contiguous segment comprises or is a contiguous segment between the higHV3-74 gene and the hIgHV3-30 gene, and the first partial hIgHV contiguous segment comprises or is a contiguous segment between the higHV4-28 gene and the higHV1-2 gene.

In some embodiments, the immunoglobulin heavy chain locus of the obtained genetically modified mouse sequentially comprises: (i) mouse Adam6a gene; (ii) mouse Adam6b gene; (iii) the contiguous segment between the higHV3-74 gene and the hIgHV3-30 gene; (iv) the contiguous segment between the higHV4-28 gene and the hIgHV1-2 gene; (v) the contiguous segment between the higHV6-1 gene and hIgHJ6 gene; (vi) the mIgHC region.

In some embodiments, in step (a), the first partial hIgHV contiguous segment, the entire hIgHD segment, and the entire hIgHJ segment of the first partial segment are inserted between the mIgHJ region and the mIgHC region by at least two steps comprising:
(a1) inserting a first contiguous segment comprising the genes from hIgHV6-1 to hIgHJ6 between the mIgHJ region and the mIgHC region; and
(a2) inserting a second contiguous segment comprising the genes from hIgHV4-28 to hIgHV1-2 into upstream of the first contiguous segment.

In some embodiments, step (a1) specifically comprises:
(a11) inserting a contiguous segment comprising genes from hIgHD6-25 to hIgHJ6 between the mIgHJ region and the mIgHC region;
(a12) inserting a contiguous segment comprising genes from hIgHD6-13 to higHD5-24 into upstream of the inserted segment of step (a11);
(a13) inserting a contiguous segment comprising genes from hIgHD1-1 to hIgHD5-12 into upstream of the inserted segment of step (a12); and
(a14) inserting a contiguous segment comprising genes from higHV6-1 to hIgHD1-1, as well as loxP and lox2272-PB5' sites, into upstream of the inserted segment of step (a13).

In some embodiments, step (a2) specifically comprises:
(a21) recombining a BAC vector comprising the second contiguous segment between the higHV4-28 and higHV1-2 genes and loxP and lox2272 sites located at both ends of the second contiguous segment in the same orientation as that in step (a14) and Cre recombinase with the genome obtained in step (a1), wherein PB3' exists between the lox2272 site and the second contiguous segment; and
(a22) contacting the recombinant genome obtained in step (a21) with PiggyBac transposase, and screening to obtain a genome comprising the first partial segment and the loxP site located upstream of the first partial segment.

In some embodiments, in step (b) described above, the second partial hIgHV contiguous segment of the second partial segment is inserted upstream of the first partial segment by at least the following steps comprising:
(b1) inserting a contiguous segment comprising genes from higHV3-74 to hIgHV3-72 between the mIgHJ region and the mIgHC region;
(b2) inserting a contiguous segment comprising genes from higHV2-70 to hIgHV1-69D, as well as PB3'-lox5171 and loxP sites, into downstream of the inserted segment of step (b1);
(b3) recombining a BAC vector comprising a contiguous segment between hIgHV1-69-2 and hIgHV3-30 genes and loxP and lox5171 located at both ends in the same orientation as that in step (b2) and the Cre recombinase with the genome obtained in step (b2), wherein PB5' exists between the lox5171 site and the contiguous segment in this step; and
(b4) contacting the recombinant genome obtained in step (b3) with the PiggyBac transposase, and screening to obtain a genome comprising the second partial segment and the loxP site located downstream of the second partial segment.

In some embodiments, step (c) comprises:
(c1) screening a Cre-positive mouse comprising the first partial segment and the second partial segment; and
(c2) mating the mouse obtained in step (c1) with a wild-type mouse, and screening a mouse not carrying Cre but comprising the first partial segment and the second partial segment, wherein the loxP site exists between the second partial segment and the first partial segment.

In some embodiments, step (d) comprises:
(d1) mating the genetically modified mouse with a wild-type mouse, and screening a positive mouse; and
(d2) mating the male and female positive mice obtained in step (d1), and screening a homozygous mouse.

Another aspect of the present invention provides a genetically modified mouse genome, wherein an immunoglobulin heavy chain locus of a genetically modified mouse sequentially comprises: (i) mouse Adam6a gene; (ii) mouse Adam6b gene; (iii) a second partial segment of a human immunoglobulin heavy chain variable region locus, comprising a second partial hIgHV contiguous segment; (iv) a first partial segment of the human immunoglobulin heavy chain variable region locus, located downstream of the second partial segment and comprising a first partial hIgHV contiguous segment, an entire hIgHD segment, and an entire hIgHJ segment, the first partial segment not comprising a segment between downstream of hIgHV1-2 gene and upstream of higHV6-1 gene; and (v) an mIgHC region.

In some embodiments, the first partial hIgHV contiguous segment of the first partial segment may start from any gene (including a functional gene, pseudogene, or ORF) in the hIgHV segment upstream of the higHV1-2 gene and terminate at the higHV1-2 gene.

In one embodiment, any gene in the hIgHV segment upstream of the hIgHV1-2 gene may be any functional gene, pseudogene, or ORF between hIgHV(III)-82 and hIgHV1-2 genes. In one embodiment, any functional gene in the hIgHV segment upstream of the higHV1-2 gene is, for example, hIgHV3-74, higHV3-73, higHV3-72, higHV2-70, higHV1-69D, higHV1-69-2, higHV2-70D, higHV1-69, higHV3-66, higHV3-64, higHV3-62, higHV4-61, higHV4-59, higHV1-58, higHV3-53, higHV5-51, higHV3-49, higHV3-48, higHV1-46, hIgHV1-45, higHV3-43, higHV4-39, higHV3-43D, higHV4-38-2, higHV3-35, higHV4-34, higHV3-33, higHV4-31, higHV3-30-5, higHV4-30-4, higHV3-30-3, higHV4-30-2, higHV4-30-1, higHV3-30, higHV4-28, higHV2-26, higHV1-24, higHV3-23D, higHV3-23, higHV3-21, higHV3-20, higHV1-18, higHV3-15, higHV3-13, higHV3-11, hIgHV5-10-1, higHV3-9, higHV3-64D, higHV3-8, higHV3-7, higHV2-5, hIgHV7-4-1, higHV4-4, or hIgHV1-3.

In one embodiment, the first partial hIgHV contiguous segment of the first partial segment starts from one of higHV3-74, higHV3-73, higHV3-72, higHV2-70, higHV1-69D, higHV1-69-2, higHV2-70D, higHV1-69, higHV3-66, higHV3-64, hIgHV3-62, higHV4-61, higHV4-59, higHV1-58, higHV3-53, higHV5-51, higHV3-49, higHV3-48, higHV1-46, higHV1-45, higHV3-43, higHV4-39, higHV3-43D, higHV4-38-2, higHV3-35, higHV4-34, higHV3-33, higHV4-31, higHV3-30-5, higHV4-30-4, higHV3-30-3, higHV4-30-2, higHV4-30-1, higHV3-30, higHV4-28, higHV2-26, higHV1-24, higHV3-23D, higHV3-23, higHV3-21, higHV3-20, higHV1-18, higHV3-15, higHV3-13, higHV3-11, hIgHV5-10-1, higHV3-9, higHV3-64D, higHV3-8, higHV3-7, higHV2-5, hIgHV7-4-1, higHV4-4, or higHV1-3, and terminates at the higHV1-2 gene, and the described genes are both contained as ends. In one embodiment, the first partial hIgHV contiguous segment of the first partial segment is a contiguous segment between the hIgHV4-28 and hIgHV1-2 genes.

In the present invention, the second partial segment is located upstream of the first partial segment, and the second partial hIgHV contiguous segment may start from any gene (including a functional gene, pseudogene, or ORF) in the hIgHV segment and terminate at any gene (including a functional gene, pseudogene, or ORF) in the hIgHV segment upstream of the start gene of the first partial hIgHV contiguous segment. In some embodiments, the second partial segment, together with the first partial segment, constitutes all functional genes upstream of hIgHV1-2 and contiguous segments therebetween, i.e., contiguous segments between the higHV3-74 and higHV1-2 genes. For example, in some embodiments, the second partial hIgHV contiguous segment comprises or is a contiguous segment between the higHV3-74 gene and the hIgHV3-30 gene, and the first partial hIgHV contiguous segment comprises or is a contiguous segment between the higHV4-28 gene and the higHV1-2 gene.

In some embodiments, the first partial segment and the second partial segment are not rearranged.

In some embodiments, the immunoglobulin heavy chain locus of the obtained genetically modified mouse sequentially comprises: (i) mouse Adam6a gene; (ii) mouse Adam6b gene; (iii) the contiguous segment between the higHV3-74 gene and the hIgHV3-30 gene; (iv) the contiguous segment between the higHV4-28 gene and the hIgHV1-2 gene; (v) the contiguous segment between the higHV6-1 gene and hIgHJ6 gene; (vi) the mIgHC region.

In some embodiments, the immunoglobulin heavy chain locus of the obtained genetically modified mouse does not comprise mIgHV5-1 and an entire mIgHV segment upstream of the gene, a contiguous segment between migHD1-1 gene and mIgHJ4 gene, and mIgHD3-1, mIgHD5-1, and mIgHD1-3 genes.

In some embodiments, the fertility of the obtained genetically modified mouse is not reduced compared to a non-genetically modified wild-type mouse.

In some embodiments, the obtained genetically modified mouse is able to produce a human-mouse chimeric antibody comprising a human heavy chain variable region and a mouse constant region.

Another aspect of the present invention provides a cell, tissue, organ, or mouse comprising the mouse genome described above.

In some embodiments, the present invention provides a cell comprising the mouse genome described above, and the cell is an embryonic cell, a B cell, or a hybridoma cell.

In some embodiments, the present invention provides a tissue comprising the mouse genome described above, and the tissue is a white pulp of the spleen or a lymph node thereof.

In some embodiments, the present invention provides an organ comprising the mouse genome described above, and the organ is the spleen.

In some embodiments, the present invention provides a mouse comprising the mouse genome described above.

Another aspect of the present invention provides a method for preparing a monoclonal antibody, the method comprising:
(a) immunizing a mouse having any one of the genomes of the present invention with an antigen;
(b) isolating and producing a cell comprising the monoclonal antibody against the antigen from the mouse; and
(c) culturing the cell to obtain the monoclonal antibody.

In some embodiments, the cell in step (c) is a spleen cell, a B cell, or a hybridoma cell.

In some embodiments, the monoclonal antibody has a human heavy chain variable region and does not have a mouse heavy chain variable region. In some embodiments, the monoclonal antibody comprises a human heavy chain variable region and a mouse constant region.

Another aspect of the present invention provides use of any one of the cells, tissues, organs, or mice of the present invention in the preparation of a monoclonal antibody.

In some embodiments, the monoclonal antibody has a human heavy chain variable region and does not have a mouse heavy chain variable region. In some embodiments, the monoclonal antibody comprises a human heavy chain variable region and a mouse constant region.

The present invention provides a mouse genome with a modified heavy chain locus, which retains endogenous ADAM6 genes and their functions, and a human-mouse chimeric antibody can be produced while the fertility is maintained. The human-mouse chimeric antibody comprises a human heavy chain variable region and a mouse constant region.

### BRIEF DESCRIPTION OF THE DRAWINGS

FIGs. 1, 3, 5, 7, 9, 11, 13, 15, 17, 19, 21, 23, 25, 27, 29, 31, 33, 34, and 35 show schematic diagrams of genome changes involved in the respective modification steps.
FIGs. 2, 4, 6, 8, 10, 12, 14, 16, 18, 20, 22, 24, 26, 28, 30, 32, 34, 36, 37, 38, and 39 show the results of PCR identification of the respective modification steps.
FIGs. 40 to 42 are plots showing the usage frequency distribution of genes at the reads level based on the obtained gene usage frequency data.
FIG. 43 is a plot showing the distribution of CDR3 amino acid lengths at the Reads level based on the obtained CDR3 length frequency data.
FIG. 44 shows the Weblogo feature analysis results of the CDR3 amino acid sequence of each sample at the reads level.
FIG. 45 shows that the genetically modified mice and wild-type mice had similar immune performance.
FIG. 46 shows representative assay cases of positive hybridoma cells obtained by screening.
FIG. 47 shows several tens of positive hybridoma cells obtained by screening, 5 of which had very strong binding ability to antigen-positive cells.
FIG. 48 shows that four genetically modified homozygous mice all exhibited relatively high immune response capability.
FIG. 49 shows that the antibody sequences obtained from the genetically modified mice were all human antibody sequences and were rich in sequence diversity.

### DETAILED DESCRIPTION

### Definitions

"higHV" in the present invention refers to the V region of a human immunoglobulin heavy chain variable region locus; when used independently, hIgHV refers to the entire V region of the human immunoglobulin heavy chain variable region locus; and when followed by a specific gene number, for example, "higHV3-30" refers to the 3-30 gene located in the V region of the human immunoglobulin heavy chain variable region locus. Similarly, "higHD" and "higHJ" are also used herein to refer to the D and J regions, respectively, of the human immunoglobulin heavy chain variable region locus.

"mIgHV" in the present invention refers to the V region of a mouse immunoglobulin heavy chain variable region locus; when used independently, mIgHV refers to the entire V region of the mouse immunoglobulin heavy chain variable region locus; and when followed by a specific gene number, for example, "mIgHV5-1" refers to the 5-1 gene located in the V region of the mouse immunoglobulin heavy chain variable region locus. Similarly, "mIgHD" and "mIgHJ" are also used herein to refer to the D and J regions, respectively, of the mouse immunoglobulin heavy chain variable region locus.

"Contiguous segment" refers to an uninterrupted nucleotide fragment between two end-point genes specified, which comprises functional genes, pseudogenes, ORFs, and other nucleotide sequences (e.g., spacer sequences) located between the two end-point genes. The term "contiguous segment between A gene and B gene" is meant to comprise the A gene, the B gene, and a contiguous gene segment therebetween. The term "upstream of A gene" or "downstream of A gene" does not comprise the A gene itself.

### Examples

### I. ES Cell Line I-Based Mouse Construction

1. Inserting an about 20-kb human gene sequence (comprising IGHJ1-6, IGHD7-27, IGHD1-26, IGHD6-25, and all gene spacer sequences) between the J and C regions of mice

The constructed vector was electroporated into wild-type ES cells, the vector had Neo resistance, and the cells were subjected to G418 drug screening. Related ES clones were selected for culture and amplification, and then subjected to PCR typing identification.

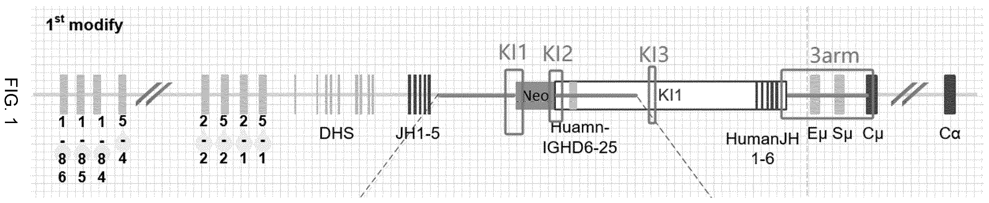

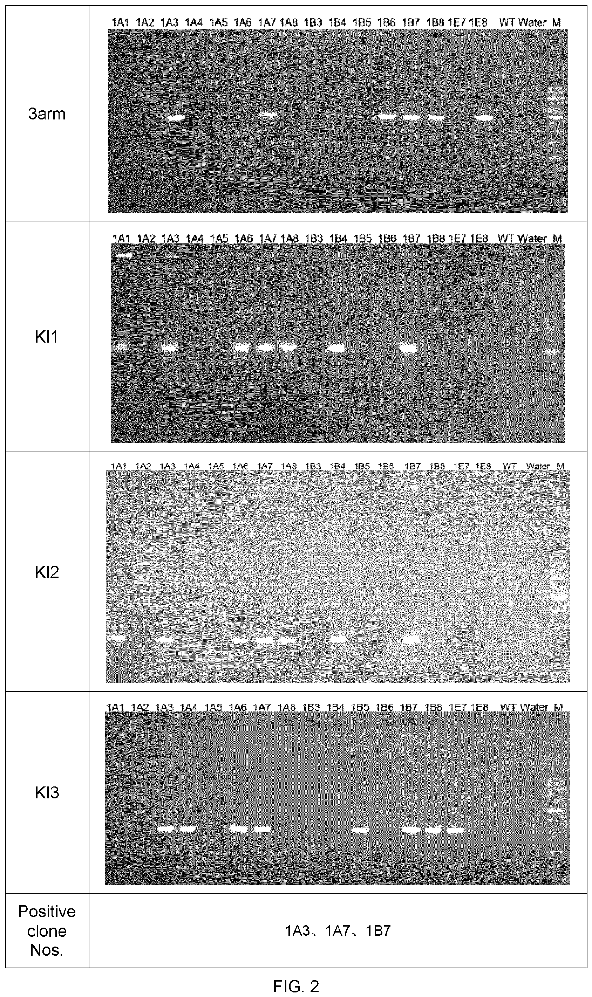

The insertion of the sequence into the corresponding position was identified by 4 pairs of primers (the sequences are shown in Table 1 below; 5'→3', the same applies hereinafter; the positions identified by the primers are also marked on the map (FIG. 1)). There were 3 clones with positive bands amplified simultaneously by the 4 pairs of primers (FIG. 2): 1A3, 1A7, and 1B7, and the 3 clones were used as positive clones in the first step.

**Table 1.**

| Procedur e | Identificatio n region | Product size | Primer name | Primer sequence | SEQ ID NO. |
|---|---|---|---|---|---|
| ES1-V1 | 3'arm | 2868 | H1-F1 | gcccatttgaaggagaggtcg | 1 |
| | | | H1-R1 | CATCTCAGCTCAGAACAGT CCAGTG | 2 |
| | KI1 | 538 | H1-F2 | acaggcagggaacagaatgtg | 3 |
| | | | H1-R2 | TGGGCTATGAACTAATGAC CC | 4 |
| | KI2 | 229 | H1-F3 | gcatcgcattgtctgagtaggt | 5 |
| | | | H1-R3 | GATGGAGCTGAGGTCTGT GC | 6 |
| | KI3 | 322 | H1-F4 | tctcaccagccacattcaag | 7 |
| | | | H1-R4 | GACACCATTTCACCTCTGT TGA | 8 |

2. Inserting an about 20-kb human D genome sequence (comprising IGHD5-24, IGHD4-23, IGHD3-22, IGHD2-21, IGHD1-20, IGHD6-19, IGHD5-18, IGHD4-17, IGHD3-16, IGHD2-15, IGHD1-14, IGHD6-13, and all gene spacer sequences) before the cell genome obtained in the first step

The constructed vector was electroporated into the positive cell clone 1A7 in the first step. The vector had Puro resistance, and a homologous arm at one end was arranged on the human sequence of the positive clone in the first step, such that the Neo resistance was deleted due to homologous recombination in this step. The cells were subjected to Puromycin drug screening, and related ES clones were selected for culture and amplification, and then subjected to PCR typing identification.

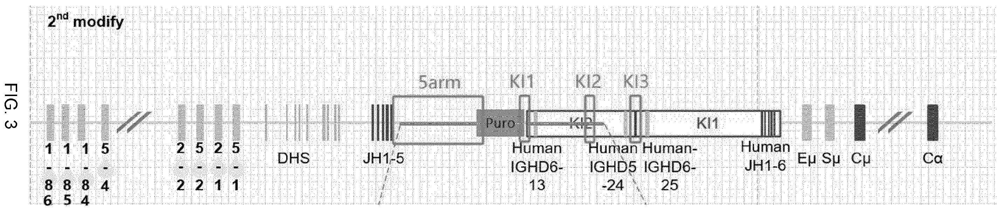

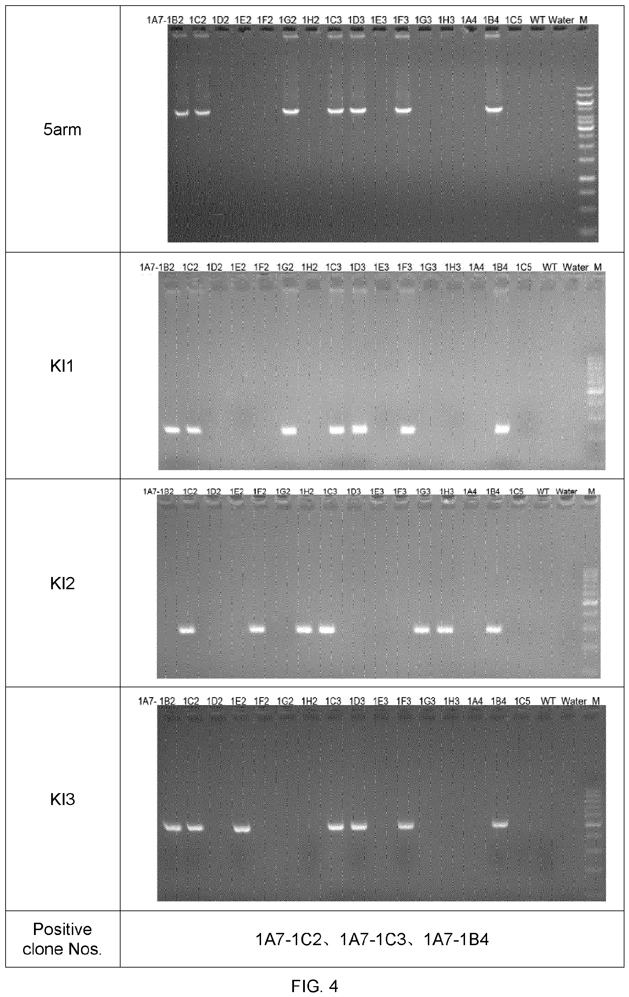

The insertion of the sequence into the corresponding position was identified by 4 pairs of primers (the sequences are shown in Table 2 below; the positions identified by the primers are also marked on the map (FIG. 3)). There were 3 clones with positive bands amplified simultaneously by the 4 pairs of primers (FIG. 4): 1A7-1C2, 1A7-1C3, and 1A7-1B4, and the 3 clones were used as positive clones in the second step.

**Table 2.**

| Procedur e | Identification region | Product size | Primer name | Primer sequence | SEQ ID NO. |
|---|---|---|---|---|---|
| ES1-V1V2 | 5'arm | 5032 | H2-F1 | gaatggacaaccaatgactggc | 9 |
| | | | H2-R1 | CGTGCTACTTCCATTTGT CACG | 10 |
| | KI1 | 232 | H2-F2 | gccttctagttgccagccatc | 11 |
| | | | H2-R2 | CCAGCCTACAGCCATAG AGCC | 12 |
| | KI2 | 282 | H2-F3 | ccagagccaactccaggagc | 13 |
| | | | H2-R3 | GCCACCAGCCAGTAGTT CTTCC | 14 |
| | KI3 | 510 | H2-F4 | cagagccagtgcagacagaggc | 15 |
| | | | H2-R4 | CTGTCCCTCTGTCTGGT GTCTGG | 16 |

3. Inserting an about 20-kb human D genome sequence (comprising IGHD5-12, IGHD4-11, IGHD3-10, IGHD3-9, IGHD2-8, IGHD1-7, IGHD6-6, IGHD5-5, IGHD4-4, IGHD3-3, IGHD2-2, IGHD1-1, and all gene spacer sequences) before the cell genome obtained in the second step

The constructed vector was electroporated into the positive cell clone 1A7-1C3 in the second step. The vector had Neo resistance, and a homologous arm at one end was arranged on the human sequence of the positive clone in the second step, such that the Puro resistance was deleted due to homologous recombination in this step. The cells were subjected to G418 drug screening, and related ES clones were selected for culture and amplification, and then subjected to PCR typing identification.

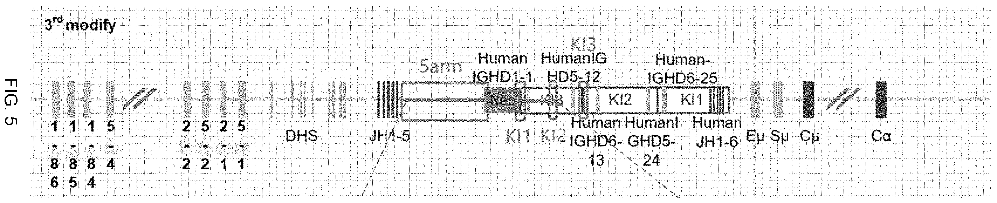

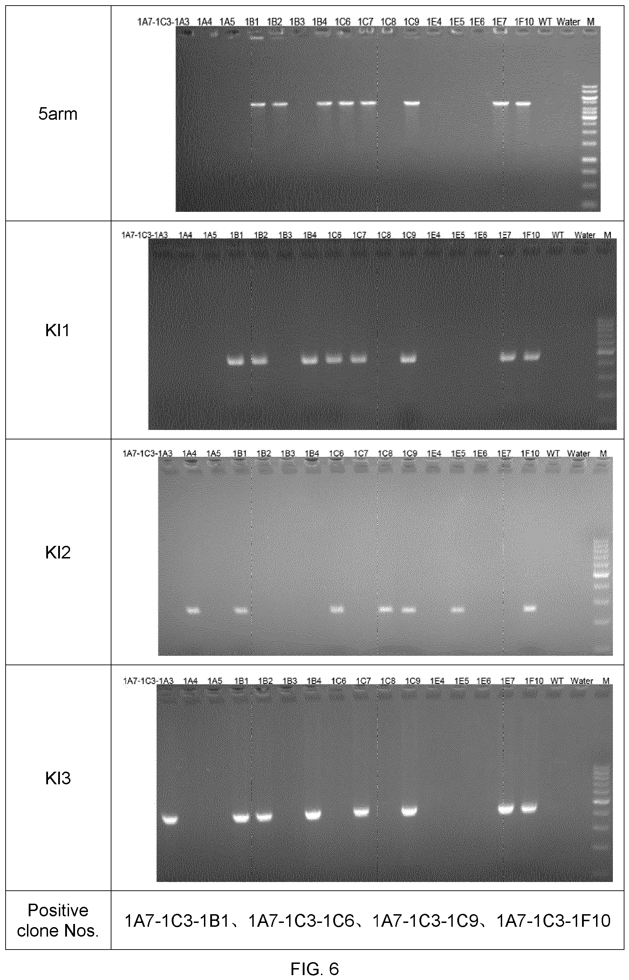

The insertion of the sequence into the corresponding position was identified by 4 pairs of primers (the sequences are shown in Table 3 below; the positions identified by the primers are also marked on the map (FIG. 5)). There were 4 clones with positive bands amplified simultaneously by the 4 pairs of primers (FIG. 6): 1A7-1C3-1B1, 1A7-1C3-1C6, 1A7-1C3-1C9, and 1A7-1C3-1F10, and the 4 clones were used as positive clones in the third step.

**Table 3.**

| Procedur e | Identification region | Product size | Primer name | Primer sequence | SEQ ID NO. |
|---|---|---|---|---|---|
| ES1-V1V2V3 | 5'arm | 4831 | H2-F1 | gaatggacaaccaatgactggc | 9 |
| | | | H1-R2 | TGGGCTATGAACTAATG ACCC | 4 |
| | KI1 | 453 | H1-F3 | gcatcgcattgtctgagtaggt | 5 |
| | | | H3-R2 | AGCGGAACACCCACAG AGAC | 17 |
| | KI2 | 256 | H3-F3 | atctgaggccgcacctgacac | 18 |
| | | | H3-R3 | CGGAACACCCACAGAG ACCGAG | 19 |
| | KI3 | 422 | H3-F4 | ggcatttctcactgtcacttct gg | 20 |
| | | | H3-R4 | CTCCTTCATGGCTCTTA GCTCC | 21 |

4. Inserting an about 20-kb human V genome sequence (comprising all spacer sequences between IGHV6-1 and upstream of IGHD1-1) before the cell genome obtained in the third step, and simultaneously introducing two lox sites loxp-lox2272

The constructed vector was electroporated into the positive cell clone 1A7-1C3-1B1 in the third step. The vector had Puro resistance, and a homologous arm at one end was arranged on the human sequence of the positive clone in the second step, such that the Puro resistance was deleted due to homologous recombination in this step, and both ends of the Puro resistance carried loxP and lox2272-PB5' (5' ITR, 5' inverted terminal repeat) elements, respectively. The cells were subjected to Puromycin drug screening, and related ES clones were selected for culture and amplification, and then subjected to PCR typing identification.

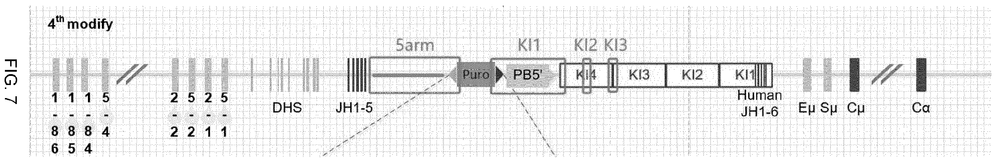

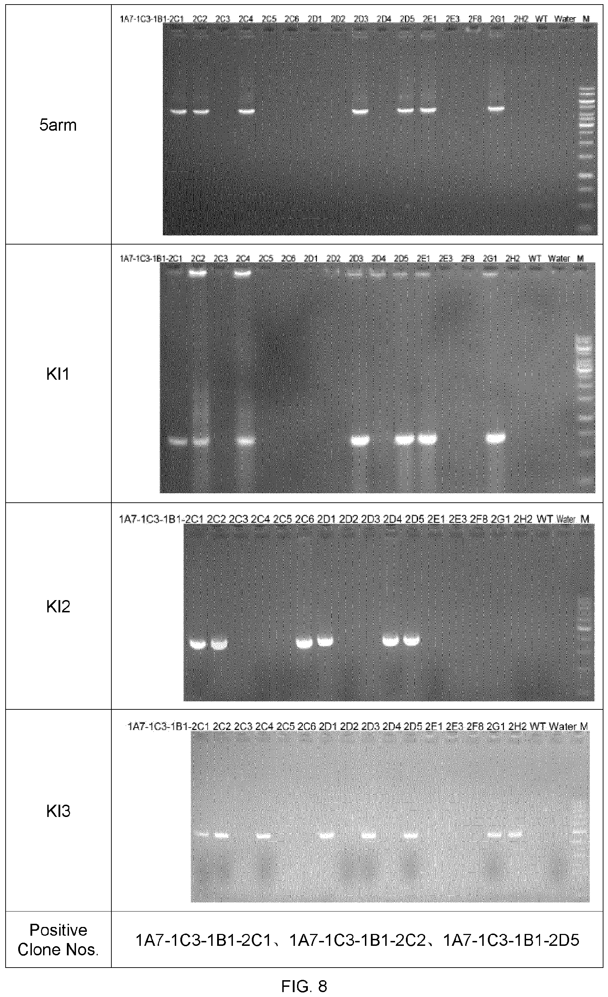

The insertion of the sequence into the corresponding position was identified by 4 pairs of primers (the sequences are shown in Table 4 below; the positions identified by the primers are also marked on the map (FIG. 7)). There were 3 clones with positive bands amplified simultaneously by the 4 pairs of primers (FIG. 8): 1A7-1C3-1B1-2C1, 1A7-1C3-1B1-2C2, and 1A7-1C3-1B1-2D5, and the 3 clones were used as positive clones in the fourth step.

**Table 4.**

| Procedure | Identification region | Product size | Primer name | Primer sequence | SEQ ID NO. |
|---|---|---|---|---|---|
| ES1-V1V2V3V4 | 5'arm | 5066 | H2-F1 | gaatggacaaccaatgactggc | 9 |
| | | | H2-R1 | CGTGCTACTTCCATTT GTCACG | 10 |
| | KI1 | 724 | H2-F2 | gccttctagttgccagccatc | 11 |
| | | | H4-R2 | AGGTCAGCATTCTGGT CCAGG | 22 |
| | KI2 | 402 | H4-F3 | cctgagagaatgatgtgctgag acc | 23 |
| | | | H4-R3 | GGAATGAGGGTTGGG TGTCCAA | 24 |
| | KI3 | 473 | H4-F4 | ggactcagcagtaaccctcagg | 25 |
| | | | H3-R2 | AGCGGAACACCCACA GAGAC | 17 |

5. Inserting an about 400-kb human V genome sequence (comprising IGHV1-2, IGHV1-3, IGHV4-4, IGHV7-4-1, IGHV2-5, IGHV3-7, IGHV3-64D, IGHV5-10-1, IGHV3-11, IGHV3-13, IGHV3-15, IGHV3-16, IGHV1-18, IGHV3-20, IGHV3-21, IGHV3-23, IGHV1-24, IGHV2-26, IGHV4-28, and all gene spacer sequences) before the cell genome obtained in the fourth step

The constructed fusion BAC and Cre were electroporated into the positive cell clone 1A7-1C3-1B1-2D5 in the fourth step. The BAC had Neo resistance (both ends of the Neo antibody carry inverted terminal repeats), both ends of the human sequence on the BAC carried loxP and lox2272 elements, respectively, in the same orientation as that of the positive clone in the fourth step, and PB3' (3' ITR, 3' inverted terminal repeat) was arranged between the lox2272 element and the human genome sequence. Under the action of Cre recombinase, the sequences between loxP and lox2272 of the BAC and the positive clone in the fourth step were replaced, and the Puro resistance was replaced by the human genome sequence and the Neo resistance on the BAC. The cells were subjected to G418 drug screening, and related ES clones were selected for culture and amplification, and then subjected to PCR typing identification.

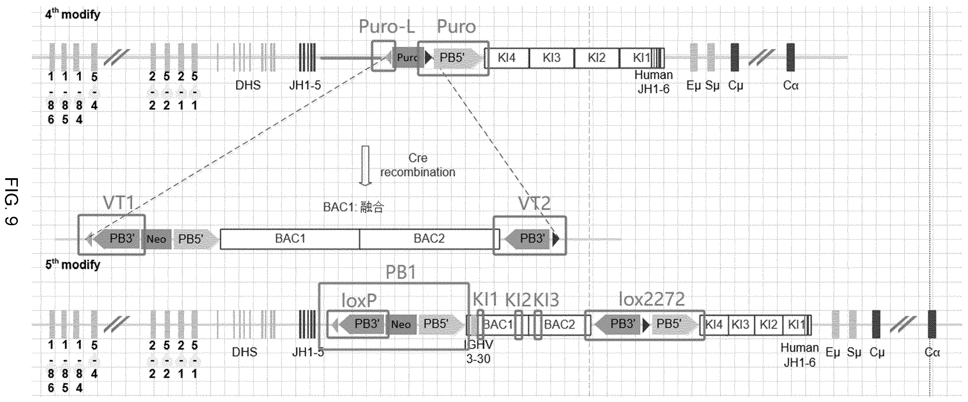

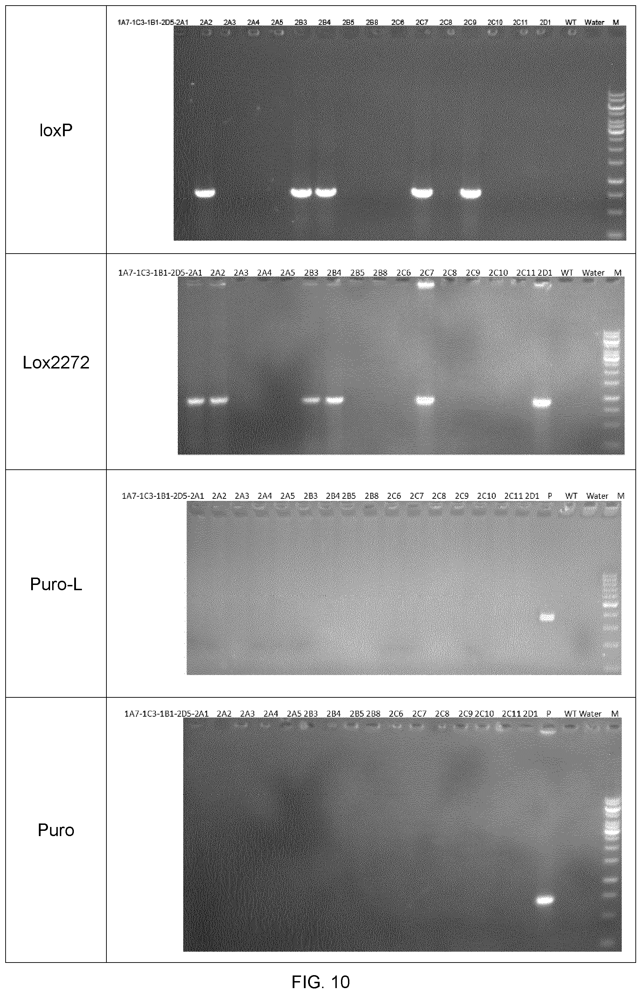

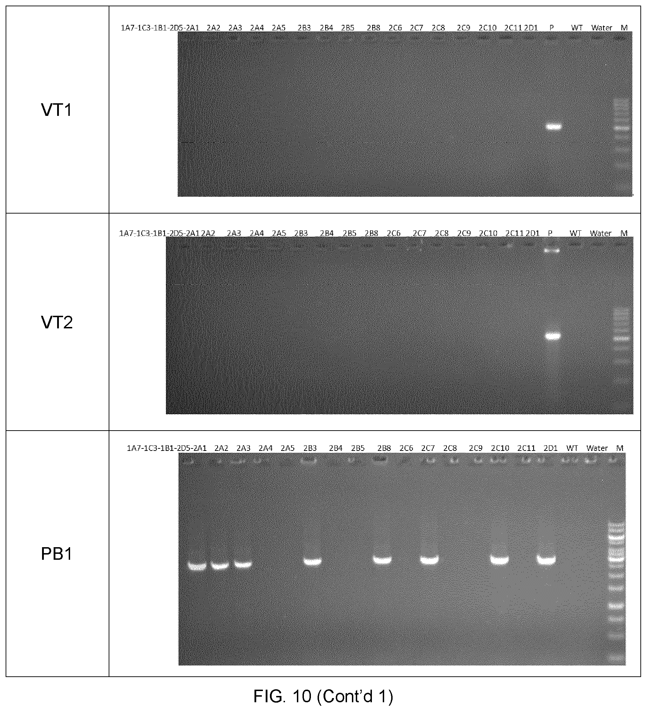

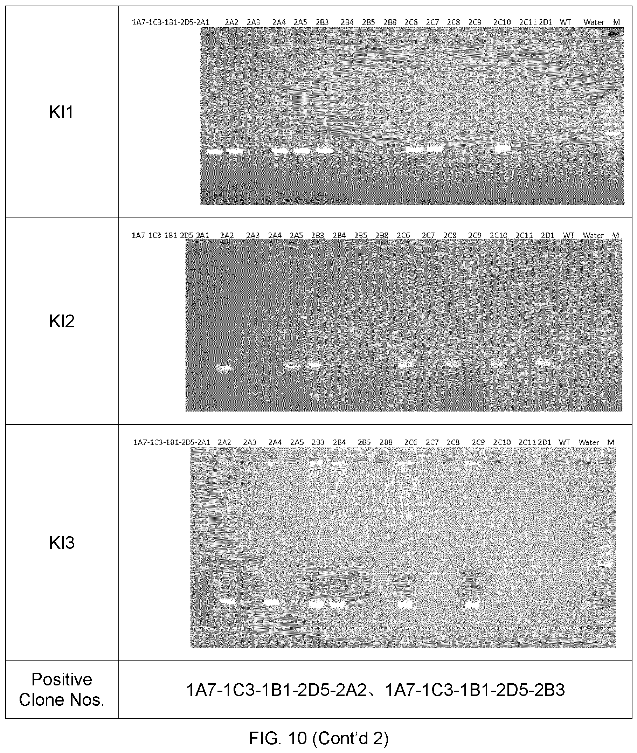

The replacement of the sequence was identified by 11 pairs of primers (the sequences are shown in Table 5 below; the positions identified by the primers are also marked on the map (FIG. 9)). There were 2 clones with positive bands amplified simultaneously by the 11 pairs of primers (FIG. 10): 1A7-1C3-1B1-2D5-2A2 and 1A7-1C3-1B1-2D5-2B3, and the 2 clones were used as positive clones in the fifth step.

**Table 5.**

| Procedur e | Identification region | Product size | Primer name | Primer sequence | SEQ ID NO. |
|---|---|---|---|---|---|
| ES1-FV+BAC | loxP | 807 | H1-F2 | acaggcagggaacagaatgtg | 3 |
| | | | H1-R2 | TGGGCTATGAACTAATGA CCC | 4 |
| | lox2272 | 889 | H4-F4 | ggactcagcagtaaccctcagg | 25 |
| | | | H4-R2 | AGGTCAGCATTCTGGTC CAGG | 22 |
| | Puro-L | 360 | H5-F3 | gatgatgggatagggactttgg | 26 |
| | | | H2-R1 | CGTGCTACTTCCATTTGT CACG | 10 |
| | Puro | 724 | H2-F2 | gccttctagttgccagccatc | 11 |
| | | | H4-R2 | AGGTCAGCATTCTGGTC CAGG | 22 |
| | VT1 | 508 | H5-F5 | gtgataataagcggatgaatgg | 27 |
| | | | H1-R2 | TGGGCTATGAACTAATGA CCC | 4 |
| | VT2 | 574 | H4-F4 | ggactcagcagtaaccctcagg | 25 |
| | | | H5-R6 | GTGGATTGCTGCTGTGT CCTG | 28 |
| | PB1 | 2930 | H1-F2 | acaggcagggaacagaatgtg | 3 |
| | | | H5-R7 | CTCTTGTTCTGTAATTCC TCCTTCC | 29 |
| | PB2=lox2272 | 889 | H4-F4 | ggactcagcagtaaccctcagg | 25 |
| | | | H4-R2 | AGGTCAGCATTCTGGTC CAGG | 22 |
| | KI1 | 361 | H5-F9 | caagatggagtctgcctgcttc | 30 |
| | | | H5-R9 | AGACGTGGTGAACCGAG CTGT | 31 |
| | KI2 | 289 | H5-F10 | ccgtgtattactttgagggacac | 32 |
| | | | H5-R10 | CCTCATAGTGTCCTGAG CGC | 33 |
| | KI3 | 224 | H5-F11 | ccttacccacactatctcttgtgtcc | 34 |
| | | | H5-R11 | CAGGCTCCCAGGTGCTC TAATTC | 35 |

### 6. Electroporating pBase (PiggyBac transposase) on the cell clone obtained in the fifth step

pBase (PiggyBac transposase) was electroporated into the positive cell clone 1A7-1C3-1B1-2D5-2B3 in the fifth step. Under the action of the PiggyBac transposase, two inverted terminal repeats were cut off from the genome, the Neo sequence and lox2272 were lost, and the positive cell clone only carried the inserted human gene sequence and one loxP element. ES clones were selected for culture and amplification, and then subjected to PCR typing identification.

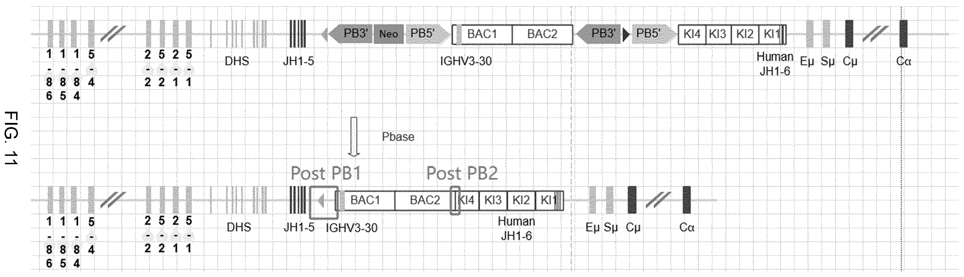

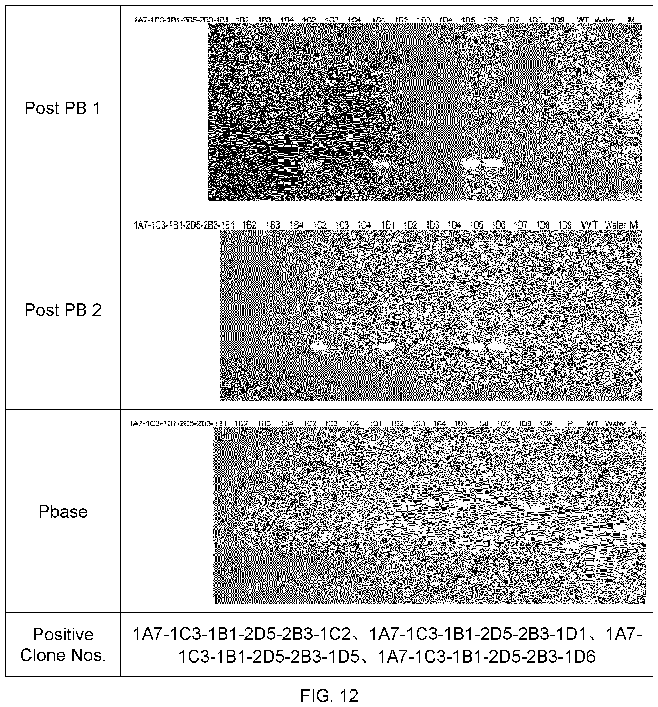

The deletion of the sequence was identified by 2 pairs of primers (the sequences are shown in Table 6 below; the positions identified by the primers are also marked on the map (FIG. 11)). There were 4 clones with positive bands amplified simultaneously by 2 pairs of primers (FIG. 12): 1A7-1C3-1B1-2D5-2B3-1C2, 1A7-1C3-1B1-2D5-2B3-1D1, 1A7-1C3-1B1-2D5-2B3-1D5, and 1A7-1C3-1B1-2D5-2B3-1D6, and the 4 clones were used as positive clones in the sixth step.

**Table 6.**

| Procedure | Identificatio n region | Produc t size | Primer name | Primer sequence | SEQ ID NO. |
|---|---|---|---|---|---|
| ES1-FV+BAC-PB | Post PB 1 | 735 | H1-F2 | acaggcagggaacagaatgtg | 3 |
| | | | H5-R7 | CTCTTGTTCTGTAATTC CTCCTTCC | 29 |
| | Post PB 2 | 307 | H4-F4 | ggactcagcagtaaccctcagg | 25 |
| | | | H4-R2 | AGGTCAGCATTCTGGT CCAGG | 22 |
| | pbase | 352 | pbase-F1 | CTGGACGAGCAGAAC GTGATCG | 36 |
| | | | pbase-R1 | CGAAGAAGGCGTAGAT CTCGTCCTC | 37 |

### 7. F0 mice obtained from ES1

The positive cell clone obtained in the sixth step was injected into blastocysts, and the blastocysts were transplanted into surrogate female mice, enabling the mice to be born after a pregnancy period of about 20 days. The claws of young mice aged 5-7 d were cut off, and DNA was extracted. PCR typing identification was carried out to determine the genotype of the mice.

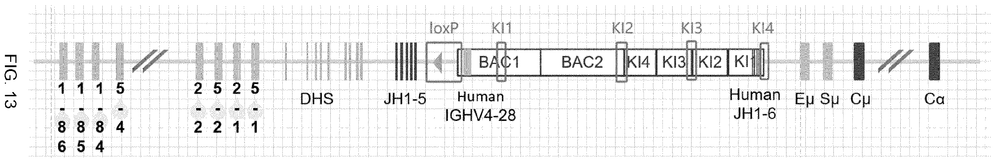

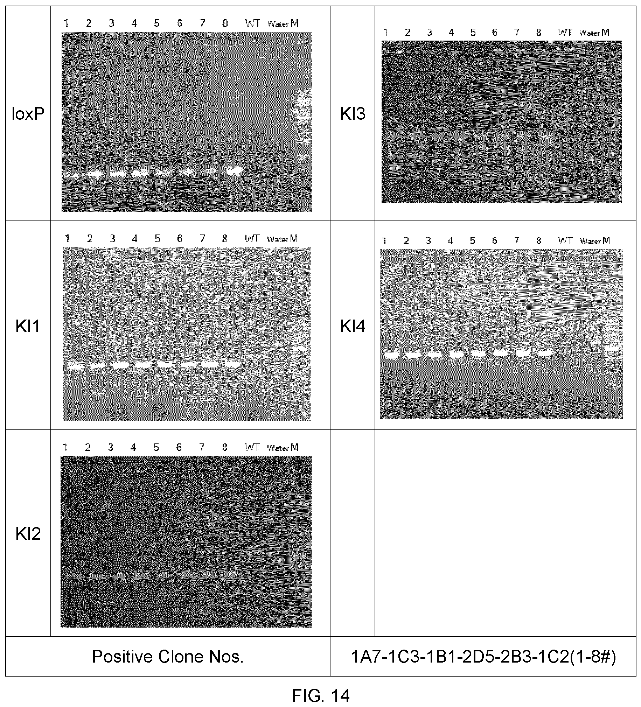

After injection of the clone 1A7-1C3-1B1-2D5-2B3-1C2, 8 mice were born in total, and identified by 5 pairs of primers (Table 7) together. The 8 mice were all positive mice (FIGs. 13 and 14).

**Table 7.**

| Procedu re | Identificatio n region | Product size | Primer name | Primer sequence | SEQ ID NO. |
|---|---|---|---|---|---|
| ES1-F0 | loxP | 735 | H1-F2 | acaggcagggaacaga atgtg | 3 |
| | | | H5-R7 | CTCTTGTTCTGTA ATTCCTCCTTCC | 29 |
| | KI1 | 361 | H5-F9 | caagatggagtctgcctg cttc | 30 |
| | | | H5-R9 | AGACGTGGTGAA CCGAGCTGT | 31 |
| | KI2 | 307 | H4-F4 | ggactcagcagtaaccct cagg | 25 |
| | | | H4-R2 | AGGTCAGCATTCT GGTCCAGG | 22 |
| | KI3 | 432 | H3-F4 | ggcatttctcactgtcacttc tgg | 20 |
| | | | H6-R4 | CCATATCTTGCTC CTTCATGGC | 38 |
| | KI4 | 460 | H1-F1 | gcccatttgaaggagagg tcg | 1 |
| | | | H6-R5 | GCAGGCTCCACC AGACCTCTC | 39 |

### 8. F1 mice obtained from ES1

The F0 mice identified as positive were mated with CMV-Cre mice to obtain F1 generation mice. The genotype of the mice was determined by PCR typing identification of the genomic DNA from the F1 generation mouse tail.

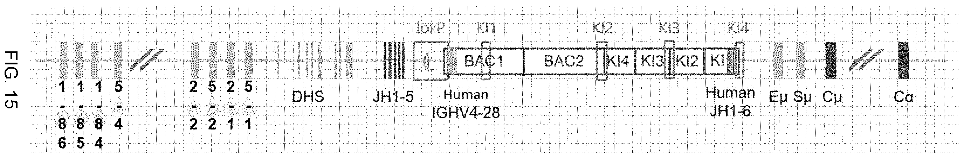

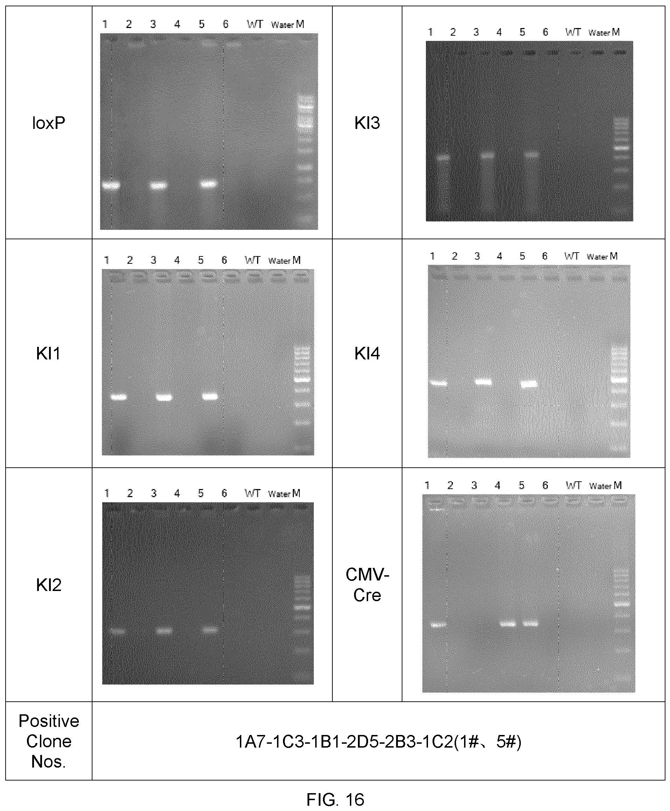

Six F1 mice were born in total. ES1 was identified by 5 pairs of primers (Table 8) together, CMV-Cre was identified by 1 pair of primers, and a total of 2 mice (1# and 5#) were double-gene-positive mice (FIGs. 15 and 16).

**Table 8.**

| Procedu re | Identificatio n region | Product size | Primer name | Primer sequence | SEQ ID NO. |
|---|---|---|---|---|---|
| ES1-F1 | loxP | 735 | H1-F2 | acaggcagggaacagaat gtg | 3 |
| | | | H5-R7 | CTCTTGTTCTGTAA TTCCTCCTTCC | 29 |
| | KI1 | 361 | H5-F9 | caagatggagtctgcctgctt c | 30 |
| | | | H5-R9 | AGACGTGGTGAAC CGAGCTGT | 31 |
| | KI2 | 307 | H4-F4 | ggactcagcagtaaccctc agg | 25 |
| | | | H4-R2 | AGGTCAGCATTCTG GTCCAGG | 22 |
| | KI3 | 432 | H3-F4 | ggcatttctcactgtcacttct gg | 20 |
| | | | H6-R4 | CCATATCTTGCTCC TTCATGGC | 38 |
| | KI4 | 460 | H1-F1 | gcccatttgaaggagaggt cg | 1 |
| | | | H6-R5 | GCAGGCTCCACCA GACCTCTC | 39 |
| | CMV-Cre | 349 | CMV-MF2 | GTAGGCGTGTACG GTGGGAGGT | 40 |
| | | | CMV-MR | TCCAGGTATGCTCA GAAAACGCC | 41 |

### II. ES Cell Line II-Based Mouse Construction

1. Inserting an about 20-kb human gene sequence (comprising IGHV3-74, IGHV3-73, IGHV3-72, and all gene spacer sequences) between the J and C regions of mice

The constructed vector was electroporated into wild-type ES cells, the vector had Neo resistance, and the cells were subjected to G418 drug screening. Related ES clones were selected for culture and amplification, and then subjected to PCR typing identification.

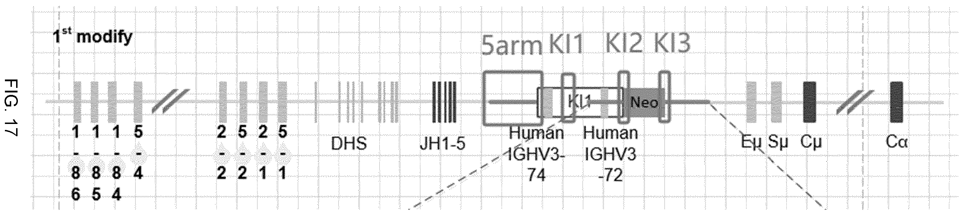

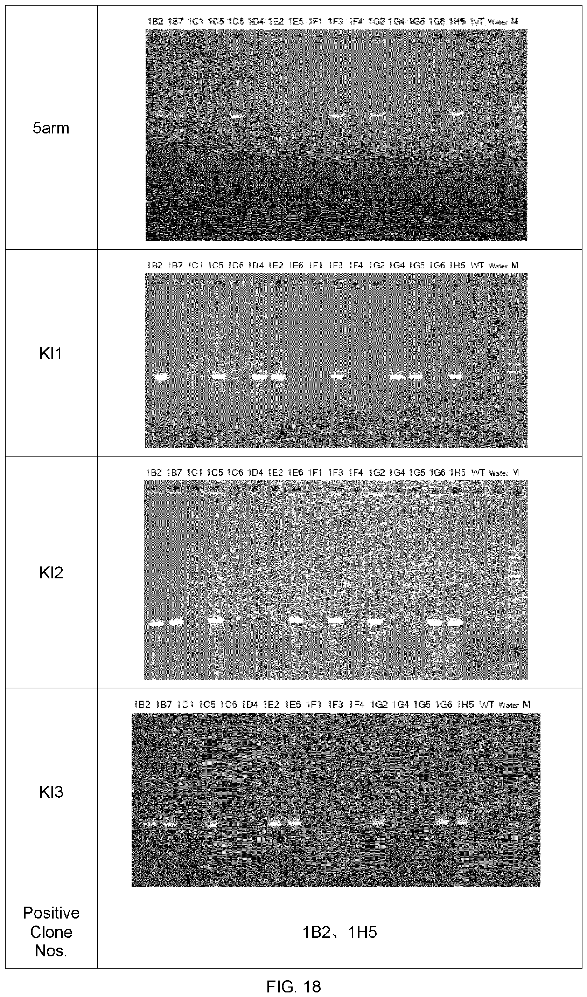

The insertion of the sequence into the corresponding position was identified by 4 pairs of primers (the sequences are shown in Table 9 below; the positions identified by the primers are also marked on the map (FIG. 17)). There were 2 clones with positive bands amplified simultaneously by the 4 pairs of primers (FIG. 18): 1B2 and 1H5, and the 2 clones were used as positive clones in the first step.

**Table 9.**

| Procedu re | Identificati on region | Product size | Primer name | Primer sequence | SEQ ID NO. |
|---|---|---|---|---|---|
| ES2-V1 | 5'arm | 4870 | H2-F1 | gaatggacaaccaatgact ggc | 9 |
| | | | H1-1R | GACTCAGCAAACGC TTCTCTGG | 42 |
| | KI1 | 420 | H1-2F | tctcaactccatcgtgacggt g | 43 |
| | | | H1-2R | GCACCACCTTCTTG TCAGTTCAGC | 44 |
| | KI2 | 895 | H1-3F | gacaggtcccaggagcagg tg | 45 |
| | | | H1-3R | AATGGAAAGTCCCT ATTGGCGTTAC | 46 |
| | KI3 | 370 | H1-4F | tgcatcgcattgtctgagtag g | 47 |
| | | | H1-4R | TTGAGACCGAGGCT AGATGCC | 48 |

2. Inserting an about 20-kb human V genome sequence (comprising IGHV2-70, IGHV1-69D, and all gene spacer sequences) into downstream of the inserted genome in the obtained cell in the first step, and simultaneously introducing two lox sites loxp-lox5171

The constructed vector was electroporated into the positive cell clone 1H5 in the first step. The vector had Puro resistance, and a homologous arm at one end was arranged on the human sequence of the positive clone in the first step, such that the Neo resistance was deleted due to homologous recombination in this step, and both ends of the Puro resistance carried PB3' (3' ITR, 3' inverted terminal repeat)-lox5171 and loxP elements, respectively. The cells were subjected to Puromycin drug screening, and related ES clones were selected for culture and amplification, and then subjected to PCR typing identification.

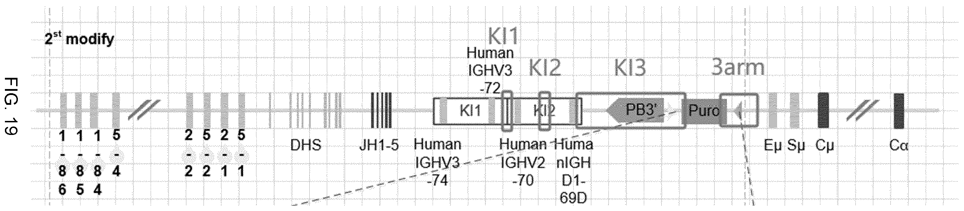

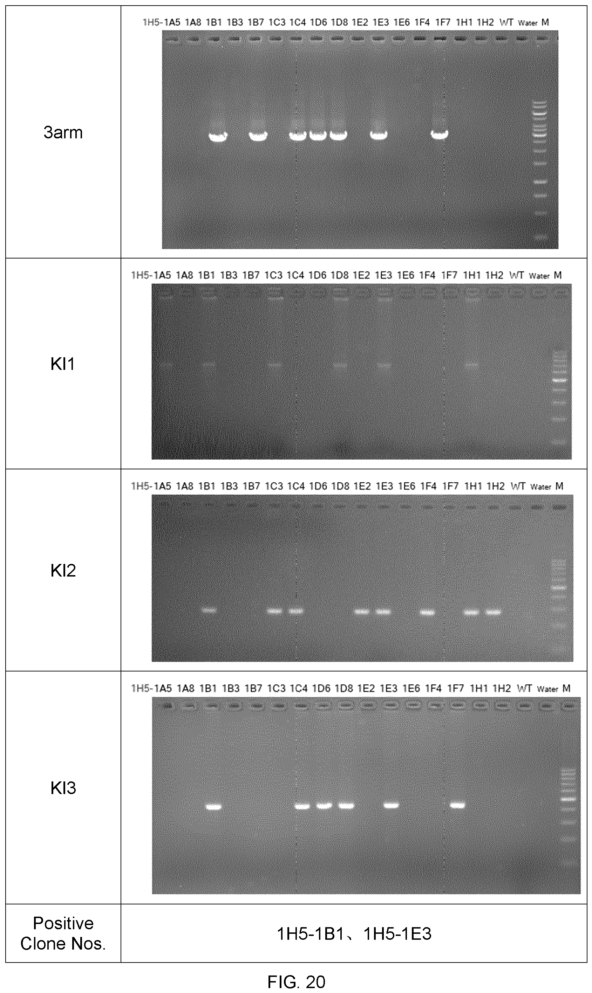

The insertion of the sequence into the corresponding position was identified by 4 pairs of primers (the sequences are shown in Table 10 below; the positions identified by the primers are also marked on the map (FIG. 19)). There were 2 clones with positive bands amplified simultaneously by the 4 pairs of primers (FIG. 20): 1H5-1B1 and 1H5-1E3, and the 2 clones were used as positive clones in the second step.

**Table 10.**

| Procedur e | Identificati on region | Product size | Primer name | Primer sequence | SEQ ID NO. |
|---|---|---|---|---|---|
| ES2-V1V2 | 3'arm | 3032 | H2-1F | cataccgacgatctgcgacc | 49 |
| | | | H1-R1 | CATCTCAGCTCAGA ACAGTCCAGTG | 2 |
| | KI1 | 690 | H2-2F | ctggagccttatgaggttgca c | 50 |
| | | | H2-2R | GGCCTGATCCAAAG AGAGGC | 51 |
| | KI2 | 277 | H2-3F | cagggaggaatctgggacc atc | 52 |
| | | | H2-3R | AATGCTGGTTACTT GTGCTTGAATT | 53 |
| | KI3 | 436 | H2-4F | agaaggtgggagcatgatg agtc | 54 |
| | | | H2-4R | GCCAGAGGCCACTT GTGTAGC | 55 |

3. Inserting a 400-kb human V genome sequence (comprising IGHV1-69-2, IGHV2-70D, IGHV1-69, IGHV3-66, IGHV3-64, IGHV4-61, IGHV4-59, IGHV1-58, IGHV3-53, IGHV8-5-51, IGHV5-51, IGHV3-49, IGHV3-48, IGHV1-46, IGHV1-45, IGHV3-43, IGHV4-39, IGHV3-38, IGHV3-35, IGHV4-34, IGHV3-33, IGHV4-31, IGHV3-30, and all gene spacer sequences) into downstream of the cell genome obtained in the second step

The constructed fusion BAC and Cre were electroporated into the positive cell clone 1H5-1B1 in the second step. The BAC had Neo resistance (both ends of the Neo resistance carry inverted terminal repeats), both ends of the human sequence on the BAC carried loxP and lox5171 elements, respectively, in the same orientation as that of the positive clone in the second step, and PB5' (5' ITR, 5' inverted terminal repeat) was arranged between the lox5171 element and the human genome sequence. Under the action of Cre recombinase, the sequences between loxP and lox5171 of the BAC and the positive clone in the second step were replaced, and the Puro resistance was replaced by the human genome sequence and the Neo resistance on the BAC. The cells were subjected to G418 drug screening, and related ES clones were selected for culture and amplification, and then subjected to PCR typing identification.

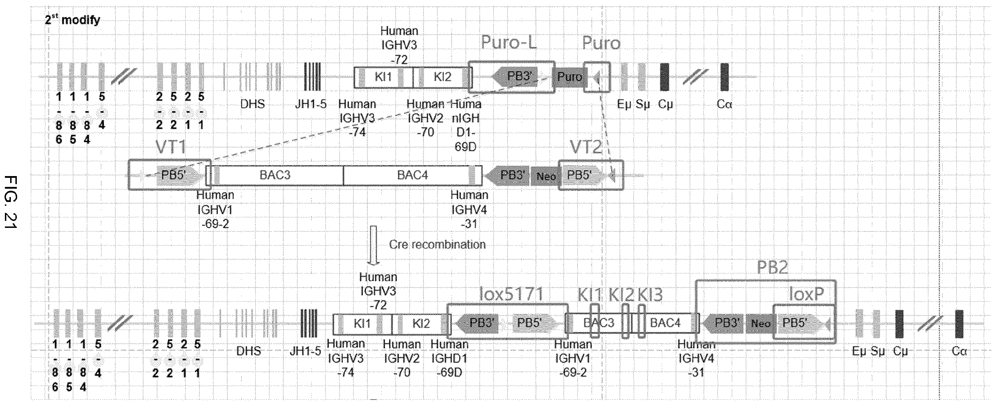

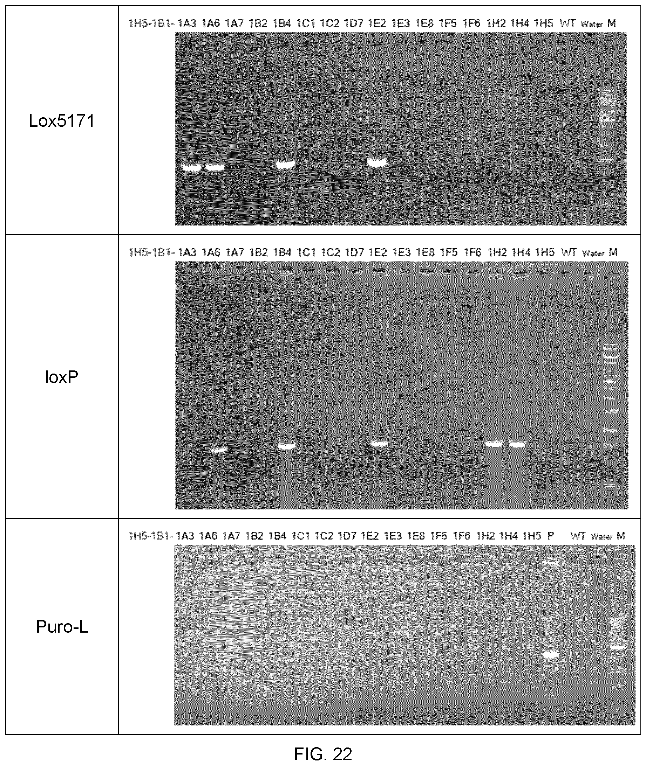

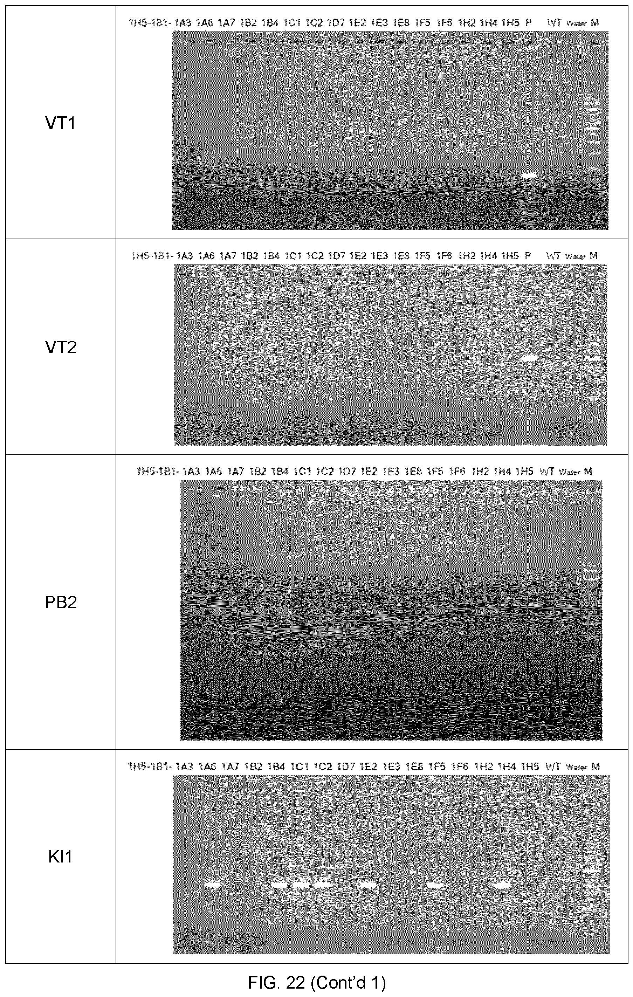

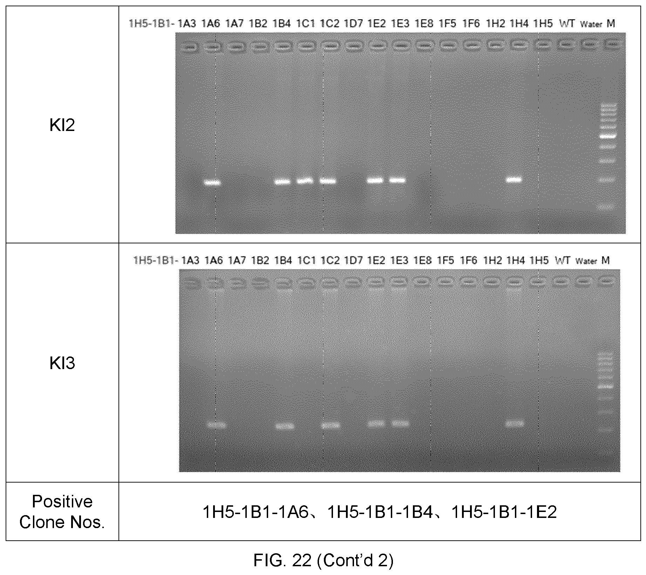

The replacement of the sequence was identified by 11 pairs of primers (the sequences are shown in Table 11 below; the positions identified by the primers are also marked on the map (FIG. 21)). There were 3 clones with positive bands amplified simultaneously by the 11 pairs of primers (FIG. 22): 1H5-1B1-1A6, 1H5-1B1-1B4, and 1H5-1B1-1E2, and the 3 clones were used as positive clones in the third step.

**Table 11.**

| Procedur e | Identification region | Produc t size | Primer name | Primer sequence | SEQ ID NO. |
|---|---|---|---|---|---|
| ES2-FV+BAC | lox5171 | 991 | H3-1F | agaaggtgggagcatgat gagtc | 56 |
| | | | H3-1R | TGGTGTCTCTTTG CGGGCTT | 57 |
| | loxP | 717 | H1-4F | tgcatcgcattgtctgagta gg | 47 |
| | | | H1-4R | TTGAGACCGAGGC TAGATGCC | 48 |
| | Puro-L | 436 | H2-4F | agaaggtgggagcatgat gagtc | 54 |
| | | | H2-4R | GCCAGAGGCCACT TGTGTAGC | 55 |
| | Puro=loxP | 391 | H1-4F | tgcatcgcattgtctgagta gg | 47 |
| | | | H1-4R | TTGAGACCGAGGC TAGATGCC | 48 |
| | VT1 | 896 | H3-5F | tggttgctacgcctgaataa gtg | 58 |
| | | | H3-1R | TGGTGTCTCTTTG CGGGCTT | 57 |
| | VT2 | 526 | H1-4F | tgcatcgcattgtctgagta gg | 47 |
| | | | H3-6R | ATGCAAGTGTGTC GCTGTCG | 59 |
| | PB1=lox517 1 | 991 | H3-1F | agaaggtgggagcatgat gagtc | 56 |
| | | | H3-1R | TGGTGTCTCTTTG CGGGCTT | 57 |
| | PB2 | 2567 | H3-8F | ggtgtgtcaagttgtcctctc cat | 60 |
| | | | H1-4R | TTGAGACCGAGGC TAGATGCC | 48 |
| | KI1 | 347 | H3-9F | catggtggtgcttgaggatt gc | 61 |
| | | | H3-9R | GAGAGCATTGATG AACACTGATGG | 62 |
| | KI2 | 204 | H3-10F | gggcctgggtagggaagt gtt | 63 |
| | | | H3-10R | CCACTGGCAGCAG TGAGATGAG | 64 |
| | KI3 | 227 | H3-11F | tgcaccaggaactcaagg aagatc | 65 |
| | | | H3-11R | CATCCAGCCTGCT TGAACTCC | 66 |

### 4. Electroporating pBase (PiggyBac transposase) on the cell clone obtained in the third step

pBase (PiggyBac transposase) was electroporated into the positive cell clone 1H5-1B1-1A6 in the third step. Under the action of the PiggyBac transposase, two inverted terminal repeats were cut off from the genome, the Neo sequence and lox5171 were lost, and the positive cell clone only carried the inserted human gene sequence and one loxP element. ES clones were selected for culture and amplification, and then subjected to PCR typing identification.

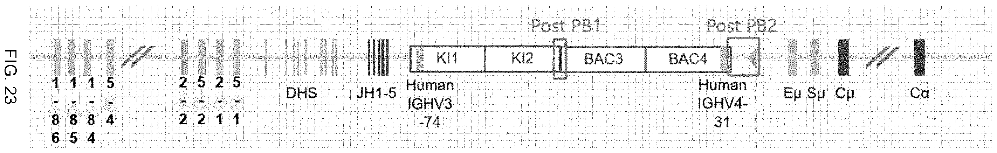

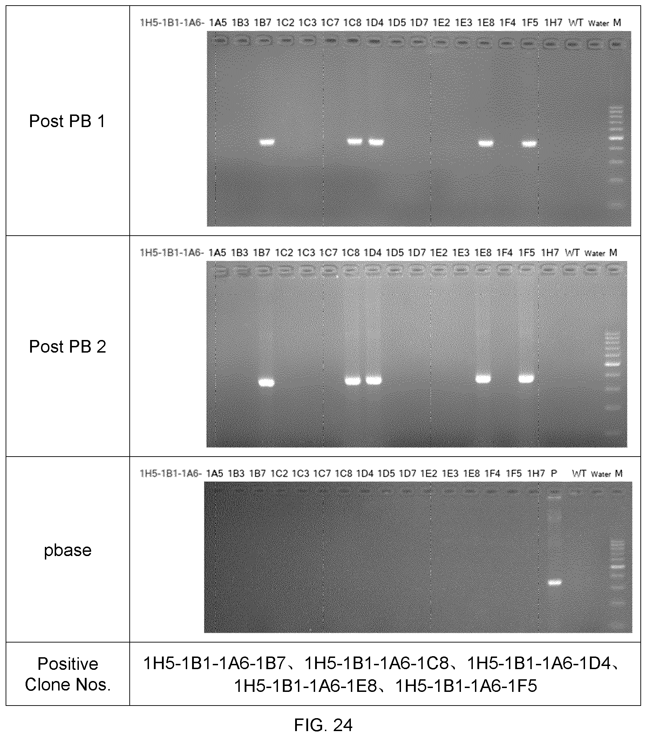

The deletion of the sequence was identified by 3 pairs of primers (the sequences are shown in Table 12 below; the positions identified by the primers are also marked on the map (FIG. 23)). There were 5 clones with positive bands amplified simultaneously by the 3 pairs of primers (FIG. 24): 1H5-1B1-1A6-1B7, 1H5-1B1-1A6-1C8, 1H5-1B1-1A6-1D4, 1H5-1B1-1A6-1E8, and 1H5-1B1-1A6-1F5, and the 5 clones were used as positive clones in the third step.

**Table 12.**

| Procedure | Identificatio n region | Produc t size | Primer name | Primer sequence | SE Q ID NO. |
|---|---|---|---|---|---|
| ES2-FV+BAC-PB | Post PB 1 | 409 | H3-1F | agaaggtgggagca tgatgagtc | 56 |
| | | | H3-1R | TGGTGTCTCTT TGCGGGCTT | 57 |
| | Post PB 2 | 372 | H3-8F | ggtgtgtcaagttgtcc tctccat | 60 |
| | | | H1-4R | TTGAGACCGA GGCTAGATGCC | 48 |
| | pbase | 352 | pbase-F1 | CTGGACGAGC AGAACGTGATCG | 36 |
| | | | pbase-R1 | CGAAGAAGGC GTAGATCTCGTCCT C | 37 |

### 5. F0 mice obtained from ES2

The positive cell clone obtained in the fourth step was injected into blastocysts, and the blastocysts were transplanted into surrogate female mice, enabling the mice to be born after a pregnancy period of about 20 days. The claws of young mice aged 5-7 d were cut off, and DNA was extracted. PCR typing identification was carried out to determine the genotype of the mice.

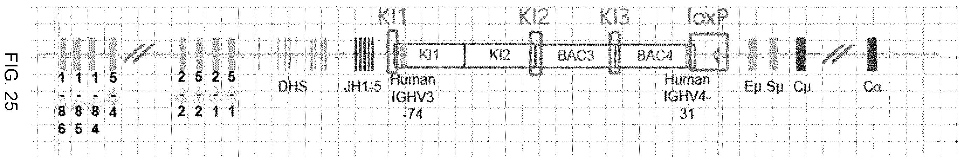

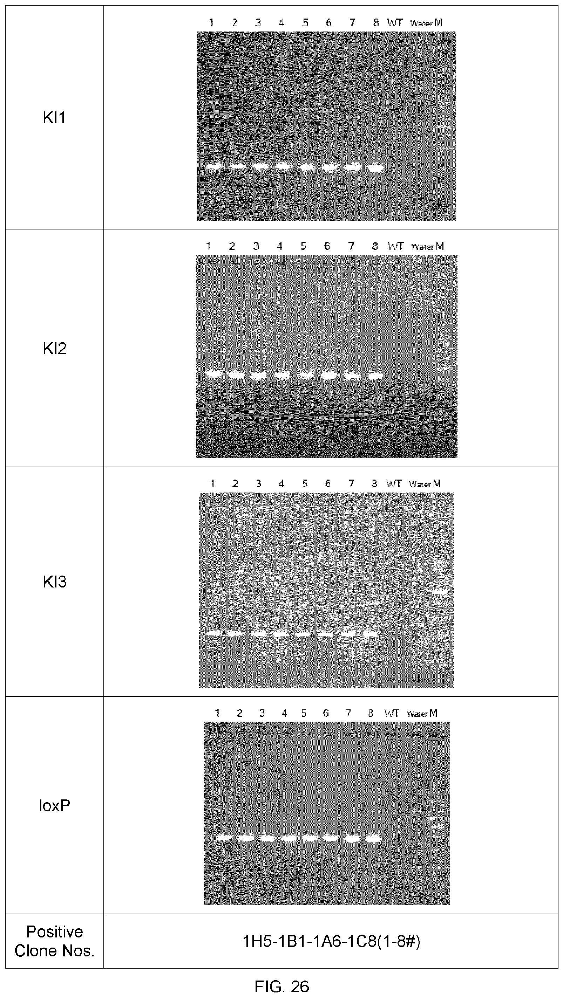

After injection of the clone 1H5-1B1-1A6-1C8, 8 mice were born in total, and identified by 4 pairs of primers (Table 13) together. The 8 mice were all positive mice (FIGs. 25 and 26).

**Table 13.**

| Procedu re | Identificatio n region | Produc t size | Primer name | Primer sequence | SEQ ID NO. |
|---|---|---|---|---|---|
| ES2-F0 | KI1 | 190 | H4-1F | gtgtgatggtgttggtgga gtcc | 67 |
| | | | H1-1R | GACTCAGCAAAC GCTTCTCTGG | 42 |
| | KI2 | 409 | H2-4F | agaaggtgggagcatga tgagtc | 54 |
| | | | H3-1R | TGGTGTCTCTTTG CGGGCTT | 57 |
| | KI3 | 204 | H3-10F | gggcctgggtagggaag tgtt | 63 |
| | | | H3-10R | CCACTGGCAGCA GTGAGATGAG | 64 |
| | loxP | 372 | H3-8F | ggtgtgtcaagttgtcctct ccat | 60 |
| | | | H1-4R | TTGAGACCGAGG CTAGATGCC | 48 |

### 6. F1 mice obtained from ES2

The F0 mice identified as positive were mated with wild-type mice to obtain F1 generation mice. The genotype of the mice was determined by PCR typing identification of the genomic DNA from the F1 generation mouse tail.

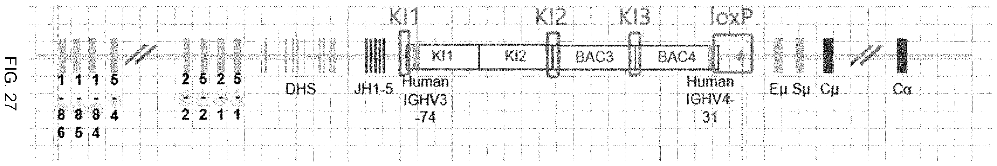

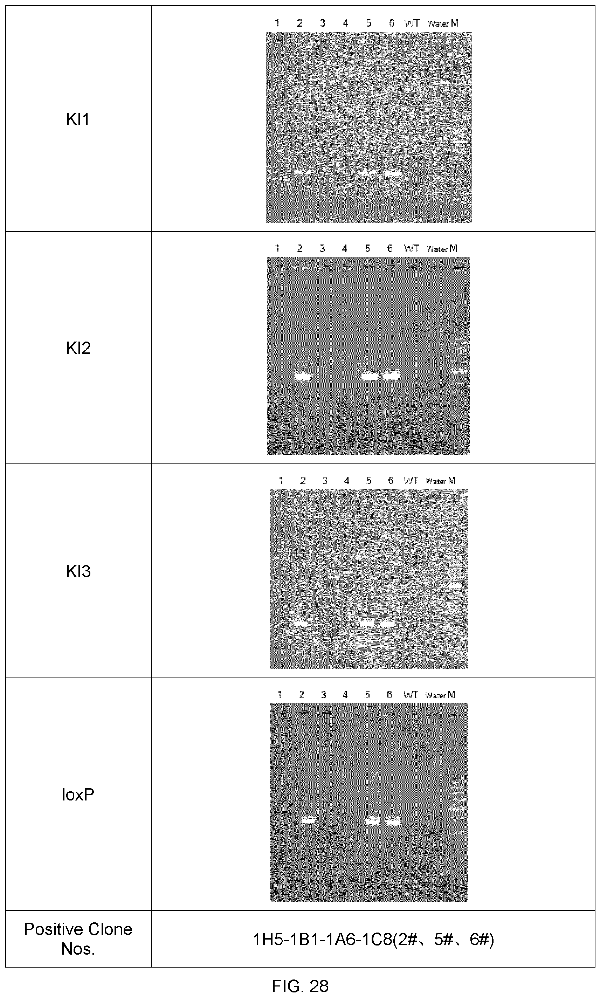

Six F1 mice were born in total. ES2 was identified by 4 pairs of primers (Table 14) together, and a total of 3 mice (2#, 5#, and 6#) were double-gene-positive mice (FIGs. 27 and 28).

**Table 14.**

| Procedu re | Identificatio n region | Product size | Primer name | Primer sequence | SEQ ID NO. |
|---|---|---|---|---|---|
| ES2-F1 | KI1 | 190 | H4-1F | gtgtgatggtgttggtggag tcc | 67 |
| | | | H1-1R | GACTCAGCAAACG CTTCTCTGG | 42 |
| | KI2 | 409 | H2-4F | agaaggtgggagcatgat gagtc | 54 |
| | | | H3-1R | TGGTGTCTCTTTG CGGGCTT | 57 |
| | KI3 | 204 | H3-10F | gggcctgggtagggaagt gtt | 63 |
| | | | H3-10R | CCACTGGCAGCAG TGAGATGAG | 64 |
| | loxP | 372 | H3-8F | ggtgtgtcaagttgtcctctc cat | 60 |
| | | | H1-4R | TTGAGACCGAGGC TAGATGCC | 48 |

Double-gene-positive F1 mice from ES1 were hybridized with F1 positive mice obtained from ES2 to obtain Cre-positive, ES1-positive, and ES2-positive F2 generation mice; the obtained F2 positive mice were mated with wild-type mice to obtain 3.5 d blastocysts; ES cell lines were established; different ES cell lines were assayed by PCR; and ectopic cells without Cre were selected, which contained the human genome sequences of ES1 and ES2 as well as a loxP sequence between IGHV3-30 and IGHV4-28.

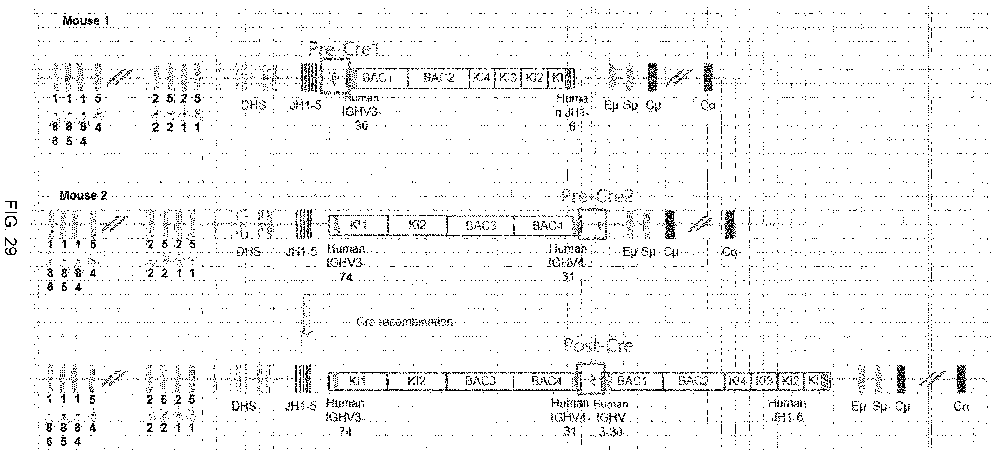

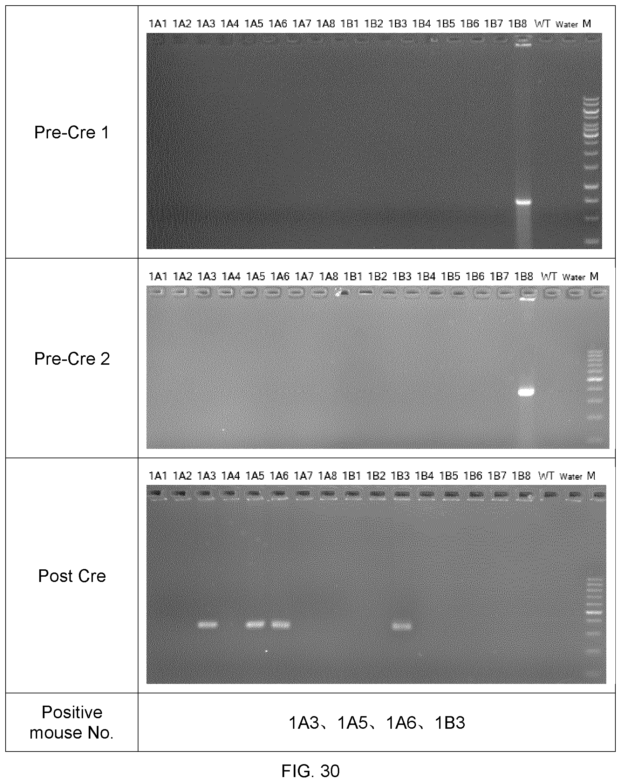

The ectopic gene was identified by 3 pairs of primers (Table 15), and there were 4 clones identified as ectopic (FIGs. 29 and 30): 1A3, 1A5, 1A6, and 1B3. The 4 clones were used as ES cell line III.

**Table 15.**

| Procedure | Identificatio n region | Prod uct size | Primer name | Primer sequence | SEQ ID NO. |
|---|---|---|---|---|---|
| ES1 + Cre F1 generation × ES2 F1 generation | Pre-Cre 1 | 735 | H1-F2 | acaggcagggaa cagaatgtg | 3 |
| | | | H5-R7 | CTCTTGTTC TGTAATTCCTCCT TCC | 2 9 |
| | Pre-Cre 2 | 372 | H3-8F | ggtgtgtcaagttg tcctctccat | 6 0 |
| | | | H1-4R | TTGAGACCG AGGCTAGATGCC | 4 8 |
| | Post Cre | 320 | Cre-F | gagcaggttcaag gctcaacc | 6 8 |
| | | | Cre-R | GAGAGGAAT CCATAACTTCGTA TAGC | 6 9 |

### III. Gene Knockout (KO)

### 1. First homologous recombination KO (V region) on ES cell line III

A vector was constructed, and the vector had Neo resistance (both ends of Neo carry recombinase sites). A homologous arm sequence at the 5 end of the vector contained a 10-kb contiguous sequence upstream of mIgHV1-86, and a homologous arm sequence at the 3 end of the vector contained an 8-kb contiguous sequence downstream of the mIgHV5-2 gene. The constructed vector was electroporated into ES cell line III. The cells were subjected to G418 drug screening, and related ES clones were selected for culture and amplification, and then subjected to PCR typing identification.

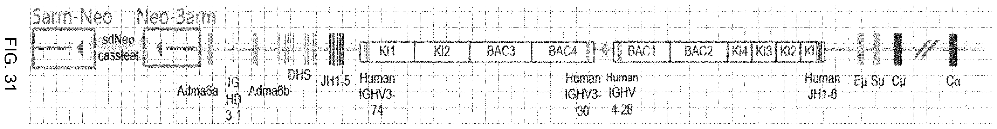

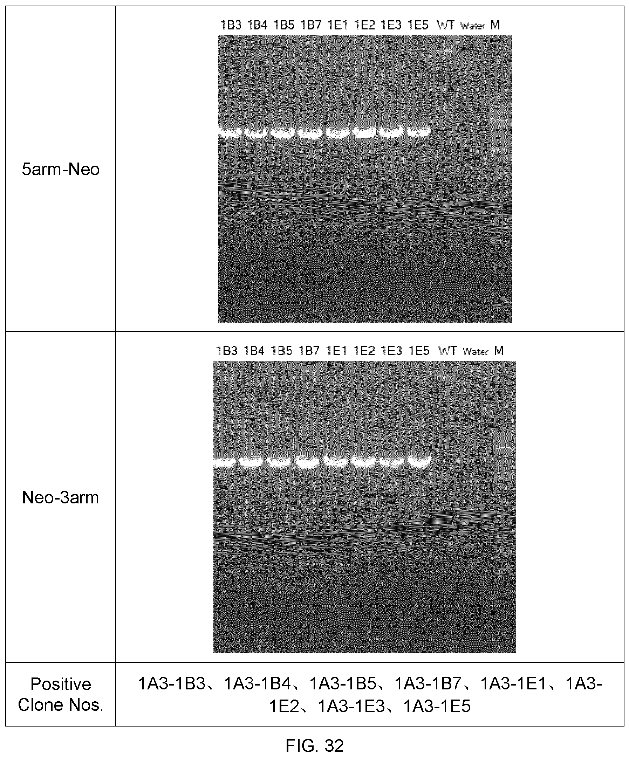

Two pairs of primers (the sequences are shown in Table 16 below; the positions identified by the primers are also marked on the map (FIG. 31)) were used to identify that the V region genes (mIgHV1-86 to mIgHV5-2) had been recombined with the Neo resistance, indicating that the mouse IgH chain V genes have been deleted by recombination. There were 8 clones with positive bands amplified simultaneously by 2 pairs of primers (FIG. 32): 1A3-1B3, 1A3-1B4, 1A3-1B5, 1A3-1B7, 1A3-1E1, 1A3-1E2, 1A3-1E3, and 1A3-1E5, and the 8 clones were used as positive clones in the first step.

**Table 16. First KO in ES cells**

| Step | Procedu re | Identification site | Size | Primer name | Primer sequence | SEQ ID NO. |
|---|---|---|---|---|---|---|
| V region KO1 | ES cells | 5arm-Neo | 4346 | H-KO1-F1 | acaaatatcc acatagaagcaa | 70 |
| | | | | H-KO1-R1 | tcaggacata gcgttggctaccc | 71 |
| | | Neo-3arm | 4 067 | H-KO1-F2 | agtgaccact gtgggcacaggggg a | 72 |
| | | | | H-KO1-R2 | ggtgagaattc tttgttcag | 73 |

### 2. Second homologous recombination KO (knockout of all gene sequences between IgHJ4 and IgHD1-1) in the cells obtained in the first step

A vector was constructed, and the vector had Puro resistance (both ends of Puro carry recombinase sites). A homologous arm sequence at the 5 end of the vector contained an 8-kb contiguous sequence upstream of mIgHD1-1, and a homologous arm sequence at the 3 end of the vector contained a 5-kb contiguous sequence downstream of the mIgHJ4 gene. The constructed vector was electroporated into the positive cell clone 1A3-1B3 in the first step. The cells were subjected to Puromycin drug screening, and related ES clones were selected for culture and amplification, and then subjected to PCR typing identification.

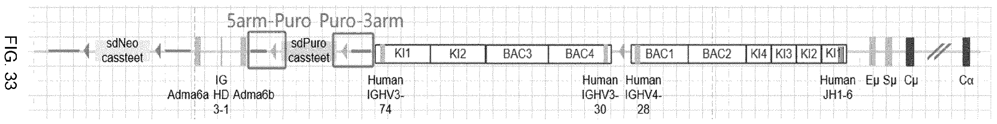

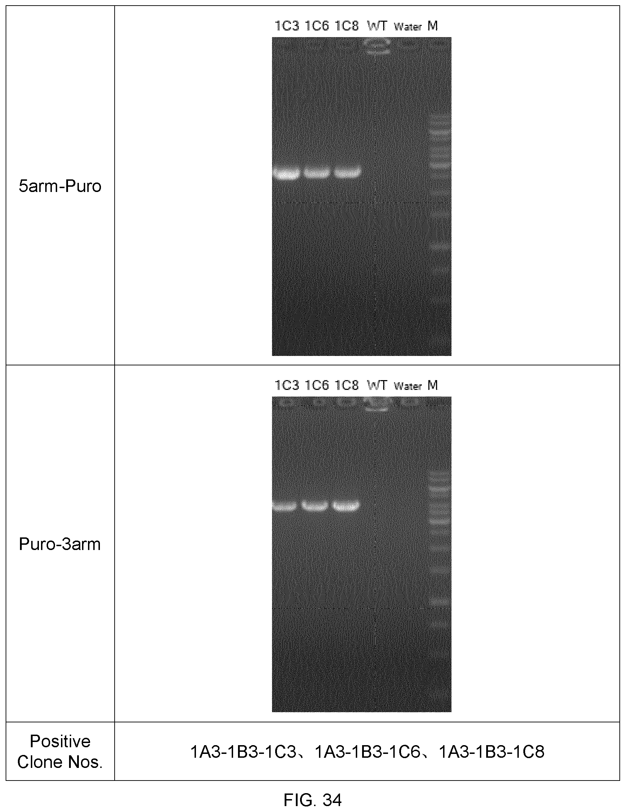

Two pairs of primers (the sequences are shown in Table 17 below; the positions identified by the primers are also marked on the map (FIG. 33)) were used to identify that the genes between mIgHJ4 and mIgHD1-1 had been recombined with the Puro resistance, indicating that the mouse IgH chain genes between mIgHJ4 and mIgHD1-1 have been deleted by recombination. There were 3 clones with positive bands amplified simultaneously by the 2 pairs of primers (FIG. 34): 1A3-1B3-1C3, 1A3-1B3-1C6, and 1A3-1B3-1C8, and the 3 clones were used as positive clones in the second step.

**Table 17. 1A3-1B3 second KO in ES cells**

| Step | Procedur e | Identificati on site | Size | Primer name | Primer sequence | SEQ ID NO. |
|---|---|---|---|---|---|---|
| DJ region KO2 | ES cells | 5arm-Puro | 2776 | H-KO2-F3 | aggtgcctgggg aatcactcag | 74 |
| | | | | H-KO2-R3 | tgtgccttctagttg ccagcc | 75 |
| | | Puro-3arm | 4016 | H-KO2-F4 | gtggaatgtgtgc gaggccag | 76 |
| | | | | H-KO2-R4 | tatgatgttagctat gaacttgt | 77 |

### 3. Third homologous recombination KO (knockout of mIgHD3-1, mIgHD5-1, and mIgHD1-3 genes) in the cells obtained in the second step

A vector was constructed, and the vector had Hygro resistance (both ends of Hygro carry recombinase sites). A homologous arm sequence at the 5 end of the vector contained an 8-kb contiguous sequence upstream of mIgHD1-3, and a homologous arm sequence at the 3 end of the vector contained a 5-kb contiguous sequence downstream of the mIgHD3-1 gene. The constructed vector was electroporated into the positive clone 1A3-1B3-1C6 in the second step. The cells were subjected to Hygromycin B drug screening, and related ES clones were selected for culture and amplification, and then subjected to PCR typing identification.

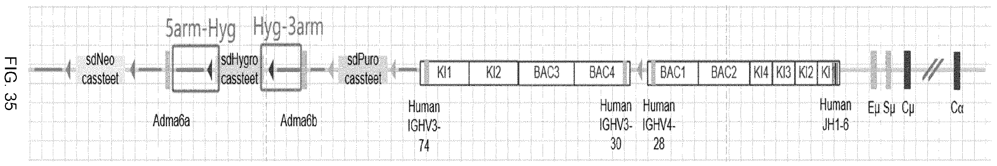

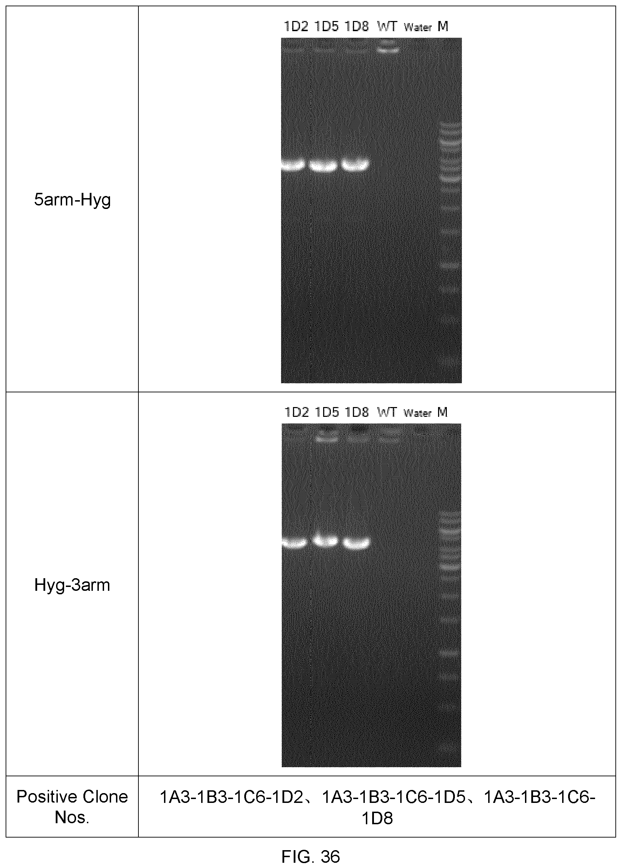

Two pairs of primers (the sequences are shown in Table 18 below; the positions identified by the primers are also marked on the map (FIG. 35)) were used to identify that the mIgHD3-1, mIgHD5-1, and mIgHD1-3 genes had been recombined with the Hygro resistance, indicating that the mouse IgH chain mIgHD3-1, mIgHD5-1, and mIgHD1-3 genes have been deleted by recombination. There were 3 clones with positive bands amplified simultaneously by the 2 pairs of primers (FIG. 36): 1A3-1B3-1C6-1D2, 1A3-1B3-1C6-1D5, and 1A3-1B3-1C6-1D8, and the 3 clones were used as positive clones in the third step.

**Table 18. 1A3-1B3-1C6 third KO in ES cells**

| Step | Proced ure | Identificati on site | Siz e | Primer name | Primer sequence | SEQ ID NO. |
|---|---|---|---|---|---|---|
| mIgHD3-1, mIgHD5-1, and mIgHD1-3 regions KO3 | ES cells | 5arm-Hyg | 377 7 | H-KO3-F5 | gtctga gccctgtgttg acagt | 78 |
| | | | | H -KO3-R5 | accctg gaaggtgcca ctc | 79 |
| | | Hyg-3arm | 431 3 | H -KO3-F6 | gacttct cttggtcccag tcca | 80 |
| | | | | H-KO3-R6 | cacaac tggcagttatc atgta | 81 |

### 4. Injecting the positive cell clone obtained in the third step to obtain positive F0 mice

The positive cell clone obtained in the third step was injected into blastocysts, and the blastocysts were transplanted into surrogate female mice, enabling the mice to be born after a pregnancy period of about 20 days. The claws of young mice aged 5-7 d were cut off, and DNA was extracted. PCR typing identification was carried out to determine the genotype of the mice.

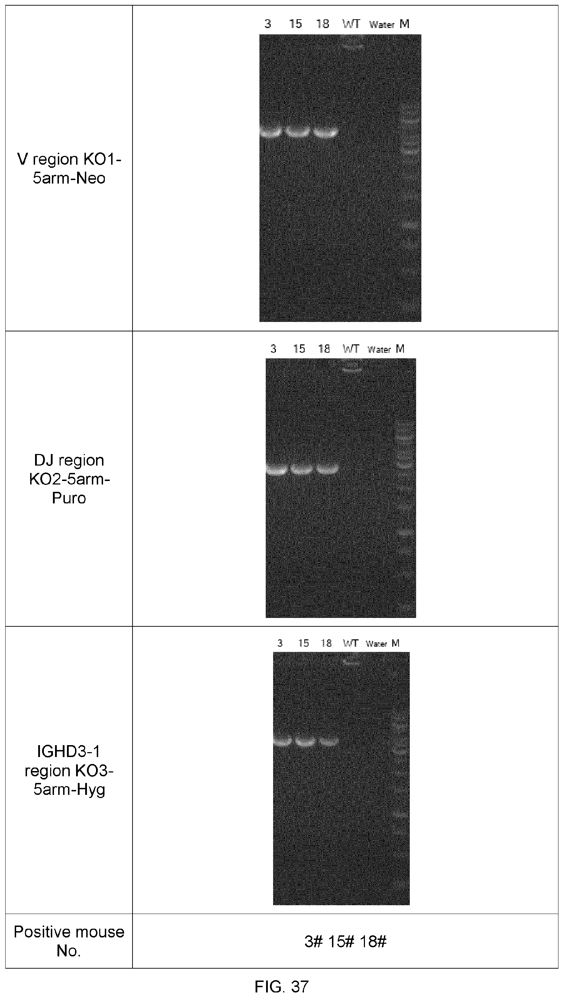

After injection of the clone 1A3-1B3-1C6-1D8, 18 mice were born in total, and identified by 3 pairs of primers (Table 19) together. 3 mice, 3#, 15#, and 18#, were all positive mice (FIG. 37).

**Table 19. Injection of post-KO ES cell-1A3-1B3-1C6-1D8 into F0 mice**

| Procedur e | Identificatio n region | Identificatio n site | Size | Primer name | Primer sequence | SEQ ID NO. |
|---|---|---|---|---|---|---|
| F0 mice | V region KO1 | 5arm-Neo | 4346 | H-KO1-F1 | acaaatatcca catagaagcaa | 70 |
| | | | | H-KO1-R1 | tcaggacatag cgttggctaccc | 71 |
| | DJ region KO2 | 5arm-Puro | 2776 | H-KO2-F3 | aggtgcctggg gaatcactcag | 74 |
| | | | | H-KO2-R3 | tgtgccttctagtt gccagcc | 75 |
| | IGHD3-1 region KO3 | 5arm-Hyg | 3777 | H-KO3-F5 | gtctgagccctg tgttgacagt | 78 |
| | | | | H-KO3-R5 | accctggaagg tgccactc | 79 |

### 5. Obtaining positive F1 mice

The F0 mice identified as positive were mated with wild-type mice to obtain F1 generation mice. The genotype of the mice was determined by PCR typing identification of the genomic DNA from the F1 generation mouse tail.

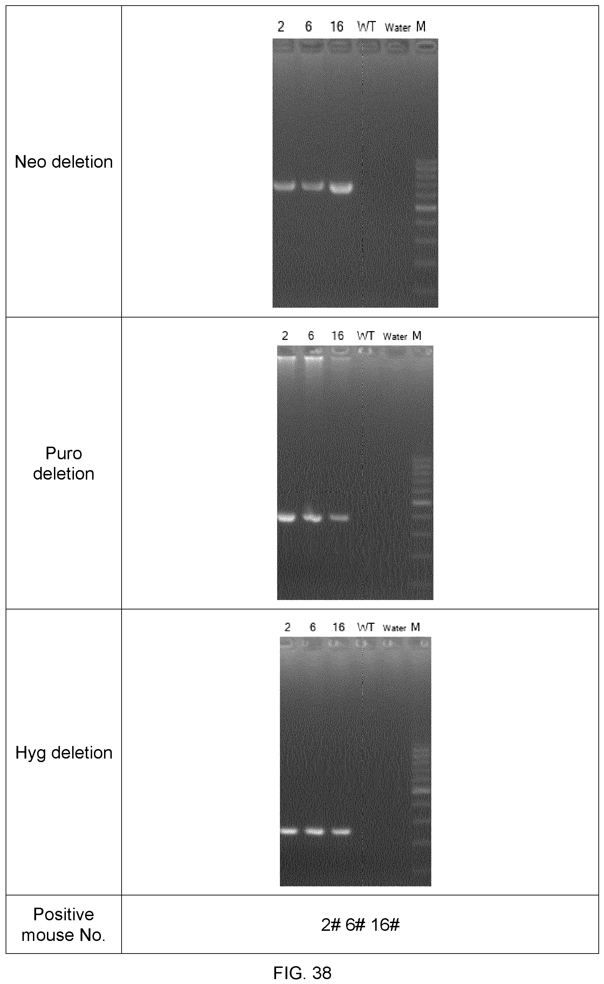

Sixteen F1 mice were born in total, and identified by 3 pairs of primers (Table 20) together. A total of 3 mice (2#, 6#, and 16#) were double-gene-positive mice (FIG. 38).

**Table 20. Mating F0-3# mice with WT to obtain F1 mice**

| Procedu re | Identification region | Identificati on site | Size | Primer name | Primer sequence | SEQ ID NO. |
|---|---|---|---|---|---|---|
| F1 mice | V region KO1 | Neo deletion | 653 | H-KO1-F | gcctctcggcctg tcaagcag | 82 |
| | | | | H-KO1-R | cagactggtgcc aggaaagca | 83 |
| | DJ region KO2 | Puro deletion | 385 | H-KO2-F | agtgctcacaca gctctgccc | 84 |
| | | | | H-KO2-R | ggtgtctgttactttt gcagaa | 85 |
| | IGHD3-1 region KO3 | Hyg deletion | 259 | H-KO3-F | agtgctcacaca gctctgccc | 86 |
| | | | | H-KO3-R | ggtgtctgttactttt gcagaa | 87 |

### 6. Obtaining homozygous mice

The male and female F1 mice identified as positive were mated to obtain homozygous mice. The genotype of the mice was determined by PCR typing identification of the genomic DNA from the F2 generation mouse tail.

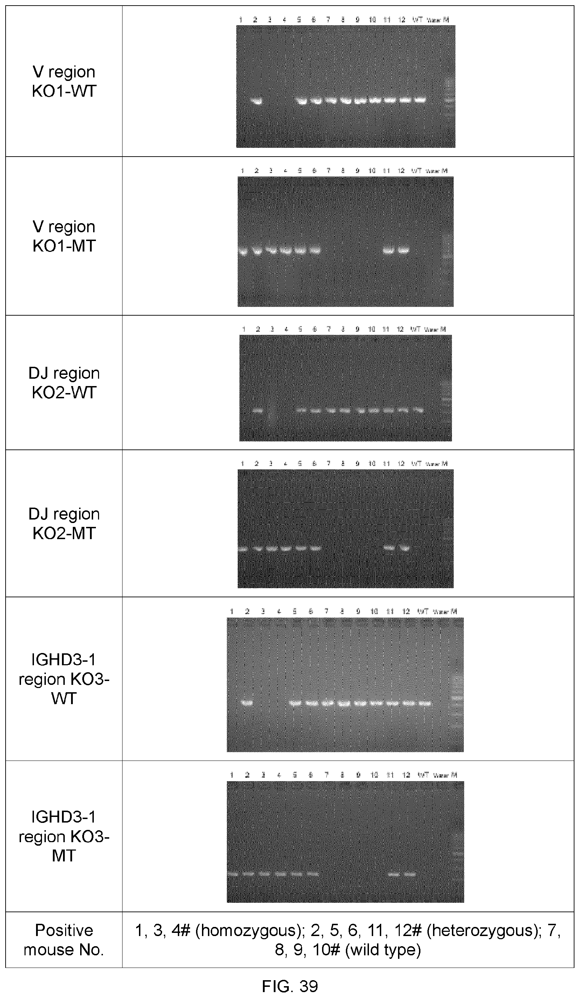

Twelve F2 mice were born in total, and identified by 6 pairs of primers (Table 21) together. The genotype of 1, 3, and 4# mice was determined to be homozygous; the genotype of 2, 5, 6, 11, and 12# mice was heterozygous; and the genotype of 7, 8, 9, and 10# mice was wild type (FIG. 39).

**Table 21. F1-2# ♂ mated with F1-16# ♀**

| Procedu re | Identificati on region | Identificati on site | Siz e | Primer name | Primer sequence | SEQ ID NO. |
|---|---|---|---|---|---|---|
| F2 mice | V region KO1 | MT | 653 | H-KO1-F | gcctctcggcctg tcaagcag | 82 |
| | | | | H-KO1-R | cagactggtgcc aggaaagca | 83 |
| | | WT | 524 | H-KO1-WT-F | ggatgctgccac attcccacct | 88 |
| | | | | H-KO1-WT-R | AAGCCATGT GAGGCAGGATC CT | 89 |
| | DJ region KO2 | MT | 385 | H-KO2-F | agtgctcacaca gctctgccc | 84 |
| | | | | H-KO2-R | ggtgtctgttactttt gcagaa | 85 |
| | | WT | 342 | H-KO2-WT-F | cttgtcagtgagg aagtccccc | 90 |
| | | | | H-KO2-WT-R | TAGCACTGT GGTAGCTACTAC C | 91 |
| | IGHD3-1 region KO3 | MT | 259 | H-KO3-F | agtgctcacaca gctctgccc | 86 |
| | | | | H-KO3-R | ggtgtctgttactttt gcagaa | 87 |
| | | WT | 407 | H-KO3-WT-F | tgggcacagctc gggctaccac | 92 |
| | | | | H-KO3-WT-R | ggtgtctgttactttt gcagaa | 93 |

### IV. Detection of Breeding Performance

The obtained homozygous mice continued to be mated, and the breeding performance of the mice was counted.

In total, 25 breeding pairs were mated, and the number of mice born after 1 month of mating was counted. There were 25 breeding pairs in total, and 20 breeding pairs gave birth to mice after 1 month of mating. The number of effective breeding pairs reached 80%, and meanwhile, the average fetal yield of the mice was 5.35, which was consistent with the fetal yield of wild-type mice. The data are shown in Table 22.

**Table 22.**

| Breeding pair | Number of births | Breeding pair | Number of births | Breeding pair | Number of births |
|---|---|---|---|---|---|
| Breeding pair 1 | 5 | Breeding pair 11 | 5 | Breeding pair 21 | 7 |
| Breeding pair 2 | 6 | Breeding pair 12 | 6 | Breeding pair 22 | 7 |
| Breeding pair 3 | 4 | Breeding pair 13 | 4 | Breeding pair 23 | 0 |
| Breeding pair 4 | 6 | Breeding pair 14 | 0 | Breeding pair 24 | 5 |
| Breeding pair 5 | 6 | Breeding pair 15 | 0 | Breeding pair 25 | 5 |
| Breeding pair 6 | 5 | Breeding pair 16 | 7 | | |
| Breeding pair 7 | 2 | Breeding pair 17 | 0 | | |
| Breeding pair 8 | 5 | Breeding pair 18 | 4 | | |
| Breeding pair 9 | 0 | Breeding pair 19 | 5 | | |
| Breeding pair 10 | 7 | Breeding pair 20 | 6 | | |

### V. H Chain Immune Repertoire Sequencing Assay

The obtained homozygous mice were euthanized and dissected to collect the spleen of the mice. RNA was extracted, and a library was constructed after the total RNA extracted from the sample was assayed to be qualified. Then, immune repertoire sequencing was performed (immune repertoire sequencing (Immuno-Seq) takes B/T lymphocytes as a research target, specifically amplifies a variable region (V region) that determines the diversity of a B cell receptor (BCR) or a T cell receptor (TCR) through 5' RACE or multiplex PCR technology, and comprehensively evaluates the diversity of an immune system in combination with high-throughput sequencing technology). Quality control was performed on the sequences obtained by sequencing using quality control software, and a sequencing background was filtered. The sequences were aligned with V, D, and J genes in an IMGT immune cell receptor library, and the corresponding gene fragments were searched to find accurate V, D, and J gene fragments and sequence sites. The statistical analysis was performed on the V, D, and J gene frequency, clone frequency distribution, the number of polypeptide sequences, and other information.

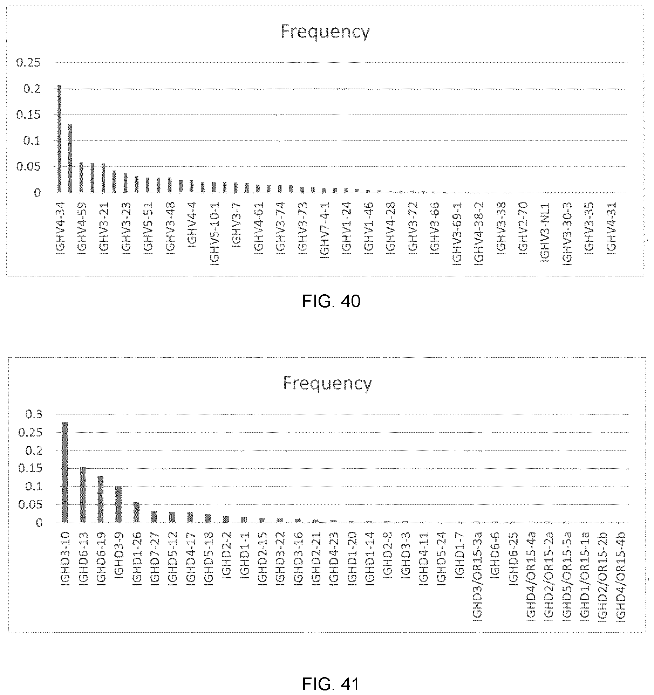

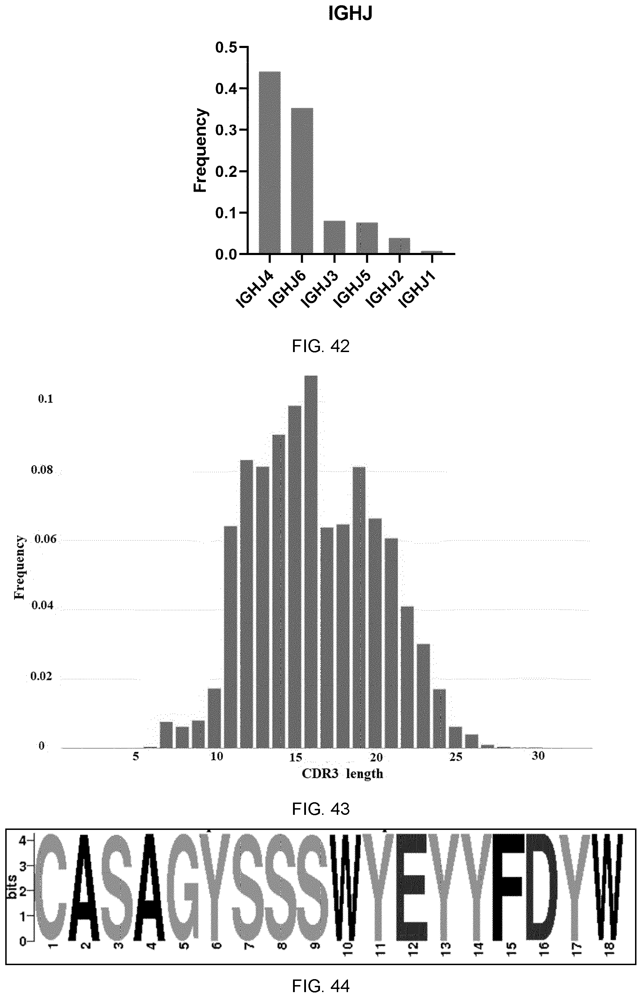

FIGs. 40 to 42 are plots showing the usage frequency distribution of genes at the reads level based on the obtained gene usage frequency data. The results in FIG. 40 show that the unimmunized homozygous mice IGHV43-34 had the highest usage frequency at the reads level, IGHV6-1 had the lower usage frequency, followed by IGHV4-59, IGHV4-39, IGHV3-21, IGHV2-26, IGHV3-23, IGHV2-5, IGHV5-51, IGHV1-18, IGHV3-48, IGHV3-15, IGHV4-4, IGHV3-43, IGHV5-10-1, IGHV3-30, IGHV3-7, IGHV1-3, IGHV4-61, IGHV3-20, IGHV3-74, IGHV1-69, IGHV3-73, IGHV1-2, IGHV7-4-1, IGHV3-64D, IGHV1-24, IGHV3-53, IGHV1-46, IGHV3-11, IGHV4-28, IGHV3-13, IGHV3-72, IGHV1-58, IGHV3-66, IGHV3-64, IGHV3-69-1, IGHV3-49, IGHV4-38-2, IGHV3-43D, IGHV3-38, IGHV3-33, IGHV2-70, IGHV3-71, IGHV3-NL1, IGHV3-22, IGHV3-30-3, IGHV3-38-3, IGHV3-35, IGHV3-52, IGHV4-31, and IGHV4/OR15-8 decreasing sequentially. The results in FIG. 41 show that the unimmunized homozygous mice IGHD3-10 had the highest usage frequency at the reads level, IGHD6-13 had the lower usage frequency, followed by IGHD6-19, IGHD3-9, IGHD1-26, IGHD7-27, IGHD5-12, IGHD4-17, IGHD5-18, IGHD2-2, IGHD1-1, IGHD2-15, IGHD3-22, IGHD3-16, IGHD2-21, IGHD4-23, IGHD1-20, IGHD1-14, IGHD2-8, IGHD3-3, IGHD4-11, IGHD5-24, IGHD1-7, IGHD3/OR15-3a, IGHD6-6, IGHD6-25, IGHD4/OR15-4a, IGHD2/OR15-2a, IGHD5/OR15-5a, IGHD1/OR15-1a, IGHD2/OR15-2b, and IGHD4/OR15-4b decreasing sequentially. The results in FIG. 42 show that the unimmunized homozygous mice IGHJ4 had the highest usage frequency at the reads level, IGHJ6 had the lower usage frequency, followed by IGHJ3, IGHJ5, IGHJ2, and IGHJ1 decreasing sequentially.

FIG. 43 is a plot showing the distribution of CDR3 amino acid lengths at the Reads level based on the obtained CDR3 length frequency data. The results indicated that the CDR3 amino acid length distribution was approximately normal.

The CDR3 amino acid sequence feature statistics is an important step of immune repertoire analysis. Different CDR3 sequences evolved from the sequences of each germline antibody in each clone are subjected to sequence abundance quantitative analysis, and finally, the sequences are conveniently selected for further antibody expression. The Weblogo feature analysis of the CDR3 amino acid sequence of each sample at the reads level is shown in FIG. 44. The results in FIG. 44 show the amino acid sequence of the highest CDR3 abundance from the unimmunized homozygous mice.

### VI. Antibody Preparation

Three C57BL/6N female mice aged 6-8 weeks and 3 HK homozygous female mice (homozygous mice in which the heavy chain variable region was subjected to heavy chain humanization modification according to the above first to third steps, and the Kappa light chain variable region was also humanized) aged 6-8 weeks were separately immunized with a stable cell strain B7-H3-CHO-K1. The immunization regimen included three conventional immunizations and one booster immunization. The three conventional immunizations were performed once every two weeks, each time by intraperitoneal injection of 2 × 10⁷ B7-H3-CHO-K1 cells into mice. Three days after the third conventional immunization, the peripheral blood of the mice was collected from the orbit, serum was collected, and the serum antibody titer of the mice was assayed by flow cytometry. The booster immunization was performed after the titer was qualified. The booster immunization was performed 10 days after the third conventional immunization by intraperitoneal injection of 2 × 10⁷ B7-H3-CHO-K1 cells into mice. Three days after the booster immunization, the spleen of the mice was collected for hybridoma fusion to prepare hybridoma cells. Positive hybridoma cell identification was performed after the hybridoma cells grew up. The supernatant was collected, and whether the supernatant contained the B7-H3 antibody was assayed by flow cytometry. The experimental results show that the immune performance of the HK homozygous mice was not inferior to that of the wild-type C57BL/6N mice, the serum antibody titers of these two types of mice were equivalent after three immunizations, and the highest titer was larger than 1:256000. Spleen cells of the HK mice were then collected and fused with myeloma SP20 cells to prepare hybridoma cells, and more than 70 B7-H3 antibody-positive hybridoma cells were obtained after multiple screening, in which the antibodies secreted by 5 hybridoma cells had very strong binding ability to antigen-positive cells.

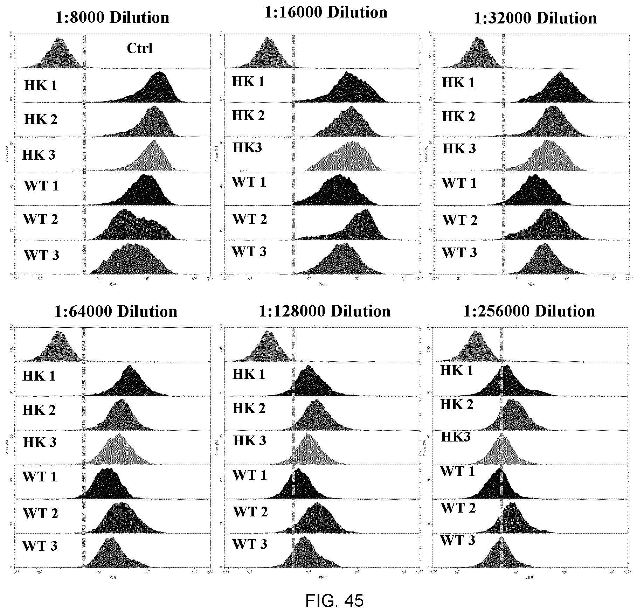

After the booster immunization, the mouse serum was collected for the antibody titer assay. The serum dilution gradient was set as follows: 1:8000, 1:16000, 1:32000, 1:64000, 1:128000, and 1:256000. The assay results in FIG. 45 show that the HK mice and the wild-type mice had similar immune performance, with both serum titers greater than 1:256000.

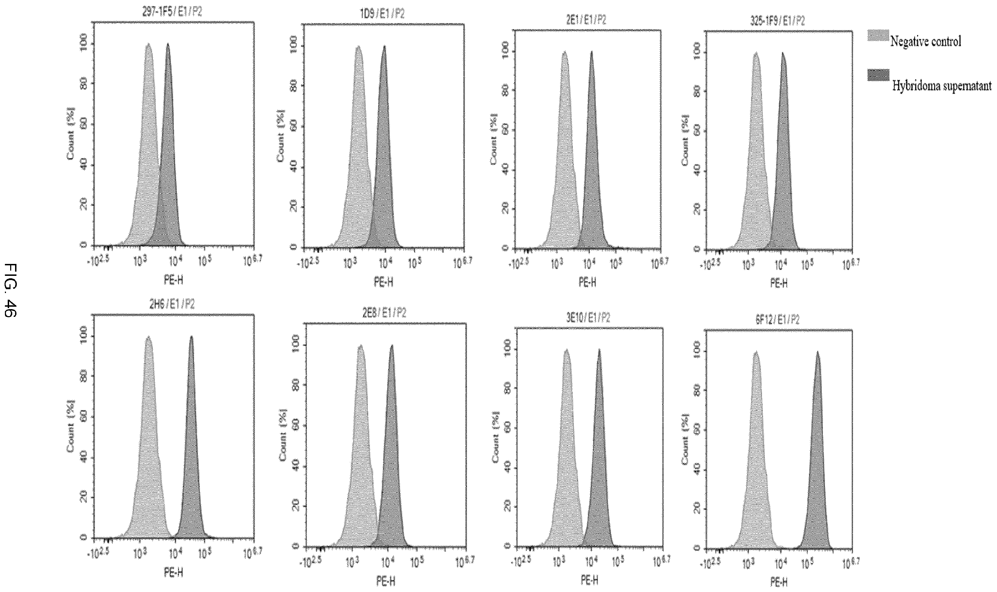

After the hybridoma cells were fused, the cell culture supernatant that was successfully fused was selected to be co-incubated with the antigen-positive cells, and the positive hybridoma cells that could secrete the antibodies were identified. FIG. 46 shows representative assay cases of positive hybridoma cells obtained by screening.

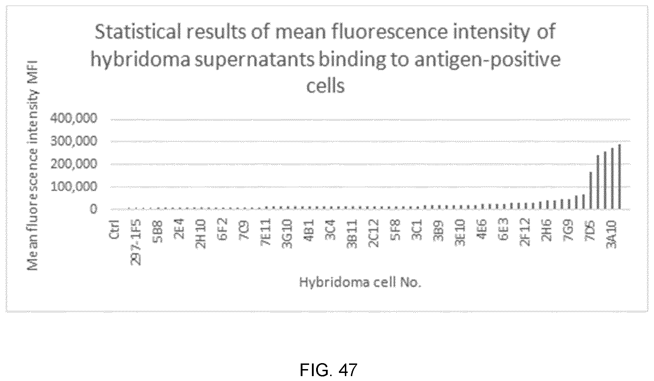

The mean fluorescence intensity of the supernatants of the positive hybridoma cells binding to the antigen-positive cells was counted. The results in FIG. 47 show that tens of positive hybridoma cells were obtained by screening, 5 of which, 5H9, 3A10, 6F12, 325-1G10, and 7D5, had very strong binding ability to the antigen-positive cells.

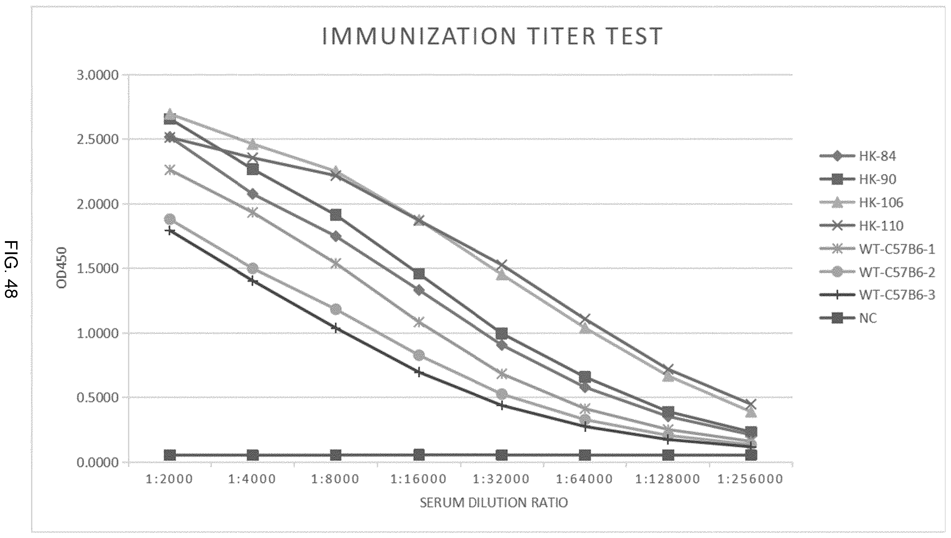

In order to generate a humoral immune response against PD-L1 in HK homozygotes (homozygous mice in which the heavy chain and Kappa light chain variable regions were humanized), complete Freund's adjuvant (CFA) and 0.05 mg of PD-L1 His (Kactus, PDL-HM110) protein were mixed and then injected subcutaneously for the first immunization, and incomplete Freund's adjuvant (IFA) and 0.025 mg of PD-L1 His protein were mixed and then injected subcutaneously. The immunization was performed once every 2 weeks for a total of 4 times, such that the HK homozygous mice generated antigen-specific antibodies. Serum titers were monitored from the HK homozygous mouse serum after the third and fourth immunizations. 2 µg/mL antigen was coated overnight with CBS (a carbonate buffer) and washed with PBST (a phosphate buffer). 2% BSA was then added, and the resulting mixture was blocked at 37 °C for 2 h. The mixture was washed with PBST, serum dilutions (serial dilution from 1:2000, 8 gradients) were then added, and the resulting mixture was blocked at 37 °C for 2 h. The mixture was washed with PBST, the horseradish peroxidase-labeled goat anti-mouse secondary antibody (diluted in a ratio of 1:1W with PBS) was then added, and the resulting mixture was incubated at 37 °C for 1 h. The mixture was washed with PBST, and the TMB substrate solution (Beyotime, P0209-500ml) was then added for color development at 37 °C for 5-10 min. A stop solution was added to stop the reaction, and the optical density was measured at 450 nm to assay the serum titer. The results are shown in FIG. 48. 4 HK homozygous mice all showed relatively high immune response capability, which reached more than 256K (HK-84, HK-90, HK-106, and HK-110 were HK homozygous mice; WT-C57B6 were wild-type mice; and NC was the serum of unimmunized mice as a negative control).

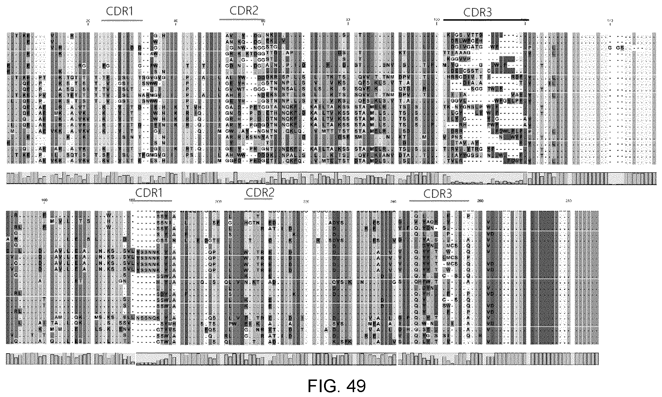

The spleens of mice with the highest serum titer after four rounds of immunization were collected, and cells were collected after grinding. The total RNA of the spleen cells was extracted using an RNA extraction kit (FOREGENE, RE-03011). cDNA was synthesized, and the antibody VH and VL sequences were amplified by using slot PCR. A vector pComb3XSS (Addgene, 63890) and a target fragment were separately subjected to enzyme digestion by Sfil and then recovered, and the ligated product was electroporated into TG1 competent cells. A PD-L1 scFv antibody library was constructed, and the library capacity was determined. The size of the library capacity was 1.36 × 109 CFM. Forty-eight clones were randomly picked from the titer plate for the number of transformants in the library for sequencing and identification, and the results (FIG. 49) show that the antibody sequences were all human antibody sequences, and the sequences were rich in diversity.

## Claims

1. A method for preparing a genetically modified mouse, comprising:
(a) inserting a first partial segment of a human immunoglobulin heavy chain variable region locus between an mIgHJ region and an mIgHC region of an immunoglobulin heavy chain locus of a first mouse, wherein the first partial segment comprises a first partial hIgHV contiguous segment, an entire hIgHD segment, and an entire hIgHJ segment, the first partial segment does not comprise a segment between downstream of hIgHV1-2 gene and upstream of higHV6-1 gene, and a first recombination site exists between upstream of the first partial segment and downstream of the mIgHJ region;
(b) inserting a second partial segment of the human immunoglobulin heavy chain variable region locus between an mIgHJ region and an mIgHC region of an immunoglobulin heavy chain locus of a second mouse, wherein the second partial segment is located upstream of the first partial segment, the second partial segment comprises a second partial hIgHV contiguous segment, and a second recombination site exists between downstream of the second partial hIgHV contiguous segment and upstream of the mIgHC region;
(c) hybridizing the first mouse with the second mouse, and screening to obtain a third mouse, wherein the second partial segment and the first partial segment located downstream of the second partial segment of the human immunoglobulin heavy chain variable region locus are inserted between an mIgHJ region and an mIgHC region of an immunoglobulin heavy chain locus of the third mouse, and a third recombination site exists between the second partial segment and the first partial segment; and
(d) knocking out mIgHV5-1 gene of the immunoglobulin heavy chain locus of the third mouse and an entire mIgHV segment upstream thereof, a contiguous segment between mIgHD1-1 gene and mIgHJ4 gene, and mIgHD3-1, mIgHD5-1, and mIgHD1-3 genes to obtain the genetically modified mouse.

2. The method according to claim 1, having one or more of the following features:
(i) the first partial hIgHV contiguous segment of the first partial segment comprises a contiguous segment between higHV4-28 gene and the higHV1-2 gene;
(ii) the second partial hIgHV contiguous segment of the second partial segment comprises a contiguous segment between higHV3-74 gene and hIgHV3-30 gene; and
(iii) the first, second, and third recombination sites are loxP sites.

3. The method according to claim 1 or 2, wherein the immunoglobulin heavy chain locus of the genetically modified mouse sequentially comprises: (i) mouse Adam6a gene; (ii) mouse Adam6b gene; (iii) the contiguous segment between the higHV3-74 gene and the hIgHV3-30 gene; (iv) the contiguous segment between the higHV4-28 gene and the hIgHV1-2 gene; (v) the contiguous segment between the higHV6-1 gene and hIgHJ6 gene; (vi) the mIgHC region.

4. The method according to any one of claims 1 to 3, wherein in step (a), the first partial hIgHV contiguous segment, the entire hIgHD segment, and the entire hIgHJ segment of the first partial segment are inserted between the mIgHJ region and the mIgHC region by at least two steps comprising:
(a1) inserting a first contiguous segment comprising the genes from higHV6-1 to hIgHJ6 between the mIgHJ region and the mIgHC region; and
(a2) inserting a second contiguous segment comprising the genes from higHV4-28 to higHV1-2 into upstream of the first contiguous segment.

5. The method according to claim 4, wherein step (a1) specifically comprises:
(a11) inserting a contiguous segment comprising genes from hIgHD6-25 to higHJ6 between the mIgHJ region and the mIgHC region;
(a12) inserting a contiguous segment comprising genes from hIgHD6-13 to hIgHD5-24 into upstream of the inserted segment of step (a11);
(a13) inserting a contiguous segment comprising genes from hIgHD1-1 to hIgHD5-12 into upstream of the inserted segment of step (a12); and
(a14) inserting a contiguous segment comprising genes from higHV6-1 to upstream of higHD1-1, as well as loxP and lox2272-PB5' sites, into upstream of the inserted segment of step (a13).

6. The method according to claim 5, wherein step (a2) specifically comprises:
(a21) recombining a BAC vector comprising the second contiguous segment between the hIgHV4-28 and higHV1-2 genes and loxP and lox2272 sites located at both ends of the second contiguous segment in the same orientation as that in step (a14) and Cre recombinase with the genome obtained in step (a1), wherein PB3' exists between the lox2272 site and the second contiguous segment; and
(a22) contacting the recombinant genome obtained in step (a21) with PiggyBac transposase, and screening to obtain a genome comprising the first partial segment and the loxP site located upstream of the first partial segment.

7. The method according to any one of claims 1 to 6, wherein in step (b), the second partial hIgHV contiguous segment of the second partial segment is inserted upstream of the first partial segment by at least the following steps comprising:
(b1) inserting a contiguous segment comprising genes from hIgHV3-74 to higHV3-72 between the mIgHJ region and the mIgHC region;
(b2) inserting a contiguous segment comprising genes from higHV2-70 to hIgHV1-69D, as well as PB3'-lox5171 and loxP sites, into downstream of the inserted segment of step (b1);
(b3) recombining a BAC vector comprising a contiguous segment between hIgHV1-69-2 and hIgHV3-30 genes and loxP and lox5171 located at both ends in the same orientation as that in step (b2) and the Cre recombinase with the genome obtained in step (b2), wherein PB5' exists between the lox5171 site and the contiguous segment in this step; and
(b4) contacting the recombinant genome obtained in step (b3) with the PiggyBac transposase, and screening to obtain a genome comprising the second partial segment and the loxP site located downstream of the second partial segment.

8. The method according to claim 7, wherein step (c) comprises:
(c1) screening a Cre-positive mouse comprising the first partial segment and the second partial segment; and
(c2) mating the mouse obtained in step (c1) with a wild-type mouse, and screening a mouse not carrying Cre but comprising the first partial segment and the second partial segment, wherein the loxP site exists between the second partial segment and the first partial segment.

9. The method according to claim 1, wherein step (d) comprises:
(d1) mating the genetically modified mouse with a wild-type mouse, and screening a positive mouse; and
(d2) mating the male and female positive mice obtained in step (d1), and screening a homozygous mouse.

10. A genetically modified mouse genome, wherein an immunoglobulin heavy chain locus of a genetically modified mouse sequentially comprises: (i) mouse Adam6a gene; (ii) mouse Adam6b gene; (iii) a second partial segment of a human immunoglobulin heavy chain variable region locus, comprising a second partial hIgHV contiguous segment; (iv) a first partial segment of the human immunoglobulin heavy chain variable region locus, located downstream of the second partial segment and comprising a first partial hIgHV contiguous segment, an entire hIgHD segment, and an entire hIgHJ segment, the first partial segment not comprising a segment between downstream of higHV1-2 gene and upstream of higHV6-1 gene; and (v) an mIgHC region.

11. The mouse genome according to claim 10, having one or more of the following features:
(i) the second partial hIgHV contiguous segment comprises or is a contiguous segment between hIgHV3-74 gene and hIgHV3-30 gene;
(ii) the first partial segment comprises or is a contiguous segment between hIgHV4-28 gene and the hIgHV1-2 gene as well as a contiguous segment between the hIgHV6-1 gene and hIgHJ6 gene located downstream;
(iii) the immunoglobulin heavy chain locus of the mouse does not comprise mIgHV5-1 gene and an entire mIgHV segment upstream thereof, a contiguous segment between mIgHD1-1 gene and mIgHJ4 gene, and mIgHD3-1, migHD5-1, and mIgHD1-3 genes;
(iv) the fertility of the mouse is not reduced compared to a non-genetically modified wild-type mouse;
(v) the first partial segment and the second partial segment are not rearranged; and
(vi) the mouse is able to produce a human-mouse chimeric antibody comprising a human heavy chain variable region and a mouse constant region.

12. A cell, tissue, organ, or mouse comprising the mouse genome according to claim 10 or 11, wherein preferably, the cell is an embryonic cell, a B cell, or a hybridoma cell; preferably, the tissue is a white pulp of the spleen or a lymph node thereof; and preferably, the organ is the spleen.

13. A method for preparing a monoclonal antibody, comprising:
(a) immunizing a mouse having the genome according to claim 10 or 11 with an antigen;
(b) isolating and producing a cell comprising the monoclonal antibody against the antigen from the mouse; and
(c) culturing the cell to obtain the monoclonal antibody;
wherein preferably, the cell is a spleen cell, a B cell, or a hybridoma cell; and preferably, the monoclonal antibody has a human heavy chain variable region and does not have a mouse heavy chain variable region.

14. Use of the cell, tissue, organ, or mouse according to claim 12 in the preparation of a monoclonal antibody, wherein preferably, the monoclonal antibody has a human heavy chain variable region and does not have a mouse heavy chain variable region; and preferably, the monoclonal antibody has a human heavy chain variable region and a mouse constant region.
